# EUROPEAN PATENT SPECIFICATION

(11) **EP 4 159 680 B1**
(45) Date of publication and mention of the grant of the patent: **29.10.2025**
(21) Application number: 21812936.9
(22) Date of filing: 24.05.2021
(51) Int. Cl.: C01F 17/235, A01N 59/16, A01P 1/00, A61K 9/10, A61K 9/14, A61K 33/244, A61P 31/12

(54) **NANOPARTICLES OF CERIUM OXIDE, DISPERSION INCLUDING NANOPARTICLES OF CERIUM OXIDE, OXIDIZING AGENT, ANTIVIRAL AGENT, AND ANTIBACTERIAL AGENT**
NANOPARTIKEL AUS CEROXID, DISPERSION MIT NANOPARTIKELN AUS CEROXID, OXIDATIONSMITTEL, ANTIVIRALES MITTEL UND ANTIBAKTERIELLES MITTEL
NANOPARTICULES D'OXYDE DE CÉRIUM, DISPERSION COMPRENANT DES NANOPARTICULES D'OXYDE DE CÉRIUM, UN AGENT OXYDANT, UN AGENT ANTIVIRAL ET UN AGENT ANTIBACTÉRIEN

(30) Priority: 25.05.2020 JP 2020090720; 19.02.2021 JP 2021025089
(43) Date of publication of application: 05.04.2023
(73) Proprietor: Toray Industries, Inc., Tokyo 103-8666 (JP)
(72) Inventor: SEKIGUCHI, Shota, Kamakura-shi, Kanagawa 248-8555 (JP); MOTOSHIROMIZU, Takahiro, Kamakura-shi, Kanagawa 248-8555 (JP); ITO, Masateru, Kamakura-shi, Kanagawa 248-8555 (JP)
(74) Representative: Kador & Partner Part mbB
(86) International application number: PCT/JP2021/019589
(87) International publication number: WO 2021/241490

(56) References cited:
- WO-A1-2006/049197
- JP-A- 2001 139 925
- JP-A- 2002 177 764
- JP-A- 2005 270 835
- JP-A- 2010 502 559
- JP-A- 2017 048 064
- JP-A- 2017 202 967
- JP-A- 2018 123 046
- JP-A- 2019 147 710
- IBRAHIM HOSSIENY ET AL: "Sensitive electrochemical sensor for simultaneous determination of uric acid and xanthine in human biological fluids based on the nano-boron doped ceria modified glassy carbon paste electrode", JOURNAL OF ELECTROANALYTICAL CHEMISTRY, ELSEVIER, AMSTERDAM, NL, vol. 780, 15 September 2016 (2016-09-15), pages 176 - 186, XP029780112, ISSN: 1572-6657, DOI: 10.1016/J.JELECHEM.2016.09.016
- M. JAFAR HUSSAIN: "Structural and Electrical Study of Boron Doped Ceria Ceramics Electrolytes for SOFC", JOURNAL OF ELECTROCHEMICAL ENERGY CONVERSION AND STORAGE, vol. 18, no. 2, 18 September 2020 (2020-09-18), pages 021007 - 1, XP093165654, ISSN: 2381-6872, DOI: https://doi.org/10.1115/1.4048279
- ZHANG J ET AL: "STRUCTURAL CHARACTERISTICS OF CERIUM OXIDE NANOCRYSTALS PREPATED BY THE MICROEMULSION METHOD", CHEMISTRY OF MATERIALS, AMERICAN CHEMICAL SOCIETY, US, vol. 13, no. 11, 10 November 2001 (2001-11-10), pages 4192 - 4197, XP001108770, ISSN: 0897-4756, DOI: 10.1021/CM010235P

## Description

### Field

The present invention relates to a cerium oxide nanoparticle, a dispersion solution containing the nanoparticle, as well as an oxidant, an antivirus agent, and an antibacterial agent; these last three agents contain the nanoparticle or the dispersion solution thereof.

### Background

Under the circumstances of increase in concerns to safety and hygiene management in recent years, an antibacterial technology to decompose a harmful substance and a microorganism is receiving an attention. For example, titanium dioxide has the property of oxidatively decomposing an organic substance through its photocatalytic characteristics, whose performance is evaluated in the degradation reaction of an organic dye. The oxidative degradation characteristics like this is expected to be used, besides the use as an antibacterial agent, also for degradation of low-molecular weight substances such as acetaldehyde and ammonia, as well as various harmful substances such as an allergen and a virus.

On the other hand, a cerium oxide nanoparticle (nanoceria) has catalytic activities similar to those of oxidative enzymes such as an oxidase and a peroxidase; thus, this is expected to be used as an oxidant. Because these catalytic activities do not require a special light source such as an ultraviolet light, the cerium oxide nanoparticle is expected to be used for the use to decompose a harmful substance even in the place where titanium oxide is difficult to be used such as indoor and in a dark place.

However, when a metal nanoparticle that is prone to readily aggregate is used as the oxidant or the like, during the synthesis thereof, a method is used to stably disperse the obtained nanoparticles by coexisting a compound that serves as a stabilizing agent. In the case of the cerium oxide nanoparticle, for example, a particle dispersion solution is obtained by oxidizing a cerium (III) ion with hydrogen peroxide using polyacrylic acid as a stabilizing agent, or by alkaline neutralization of a cerium (III) ion in an aqueous ammonia using dextran as a stabilizing agent.

In Non Patent Literature 1, a method for synthesizing the cerium oxide nanoparticle whose surface is covered with polyacrylic acid or with dextran is described. Non Patent Literature 1 especially discloses that when polyacrylic acid is used as the stabilizing agent, an oxidase activity, which is a value indicative of the oxidative performance, increases.

Also disclosed in Patent Literature 1 is an abrasive composition containing colloidal ceria whose surface is modified with boric acid, and in it, it is described that a negatively charged particle thereof can be stably dispersed over a wide pH range.

Furthermore, Patent Literature 2 discloses a reverse micellar composition having the nanoceria and boric acid dispersed in a hydrocarbon liquid or a diesel fuel, in which an organic carboxylic acid such as ethylenediaminetetraacetic acid (EDTA) or lactic acid is used as the stabilizing agent; here, it is described that when the fuel added with this composition is used, the efficiency of a diesel engine can be enhanced.

### Citation List

### Non Patent Literature

Non Patent Literature 1: A. Asati, Angew. Chem. Int. Ed. 2009, 48, 2308-2312

### Patent Literature

Patent Literature 1: Japanese Patent Application Laid-open No. 2003-183631
Patent Literature 2: Japanese Translation of PCT Application Laid-open No. 2010-502821

### Summary

### Technical Problem

The inventors of the present invention investigated the use in which the oxidative performance of the cerium oxide nanoparticle is utilized. However, the oxidative degradation of an organic dye using the cerium oxide nanoparticle whose surface is covered with polyacrylic acid as described in Non Patent Literature 1 or a commercially available cerium oxide nanoparticle resulted in a low degradation rate. The surface-modified cerium oxide nanoparticle produced by post-addition of boric acid with reference to the production method of Patent Literature 1 also resulted in a low degradation rate. Furthermore, even in the dispersion solution in which boric acid was post-added to the cerium oxide nanoparticles using EDTA/lactic acid as a stabilizing agent with reference to Patent Literature 2, the degradation rate was low. On the basis of these results, the study was further carried out with an aim to find the cerium oxide nanoparticle that has a high oxidative performance.

### Solution to Problem

In order to solve the problems mentioned above, the inventors of the present invention studied with focusing on the method for producing the cerium oxide nanoparticle, especially on the stabilizing agent. As a result, it was found that the dispersion solution containing the cerium oxide nanoparticle that is produced by adding an oxidant into a solution containing a boron compound represented by the general formula BRₙ(OR')₃₋ₙ and a cerium (III) ion could enhance the degradation rate of an organic dye. The dispersion solution produced in this way was found to have a high antivirus performance as well; the present invention was completed on the basis of these findings.

The present invention is as follows.
(1) A cerium oxide nanoparticle produced by adding an oxidant to a solution comprising a boron compound represented by following general formula (I) and a cerium (III) ion:

   BRₙ(OR')₃₋ₙ (I)

   in formula (I), n represents an integer of 0 to 2, R represents any of an alkyl group having 1 to 4 carbon atoms, a phenyl group, and a tolyl group, and R' represents any of a hydrogen atom, an alkyl group having 1 to 4 carbon atoms, a phenyl group, and a tolyl group, and when a plurality of Rs or of R's are present, the plurality of Rs or of R's are optionally same or different.
(2) The cerium oxide nanoparticle according to (1), wherein pH of the solution when the oxidant is added is 5 or more.
(3) The cerium oxide nanoparticle according to (1) or (2), wherein the boron compound represented by the general formula (I) is a boric acid, a boric acid ester, a boronic acid, a boronic acid ester, a borinic acid, a borinic acid ester, or a borate salt.
(4) The cerium oxide nanoparticle according to any one of (1) to (3), wherein 0.001 mole or more of boron relative to 1 mole of cerium element is included.
(5) The cerium oxide nanoparticle according to any one of (1) to (4), wherein the cerium oxide nanoparticle comprises the boron compound represented by the general formula (I) and has local maximum absorptions in 5726 eV to 5729 eV and in 5735 eV to 5739 eV in XANES spectrum of the cerium oxide nanoparticle.
(6) The cerium oxide nanoparticle according to any one of (1) to (5), wherein 0.0001 mole or more of a transition metal relative to 1 mole of cerium element is included.
(7) A cerium oxide nanoparticle, comprising a boron compound represented by following general formula (I), wherein
   the cerium oxide nanoparticle has local maximum absorptions in 5726 eV to 5729 eV and in 5735 eV to 5739 eV in the XANES spectrum of the cerium oxide nanoparticle:

   BRₙ(OR')₃₋ₙ (I)

   in formula (I), n represents an integer of 0 to 2, R represents any of an alkyl group having 1 to 4 carbon atoms, a phenyl group, and a tolyl group, and R' represents any of a hydrogen atom, an alkyl group having 1 to 4 carbon atoms, a phenyl group, and a tolyl group, and when a plurality of Rs or of R's are present, the plurality of Rs or of R's are optionally same or different.
(8) The cerium oxide nanoparticle according to (7), wherein the boron compound represented by the general formula (I) is a boric acid, a boric acid ester, a boronic acid, a boronic acid ester, a borinic acid, a borinic acid ester, or a borate salt.
(9) A dispersion solution comprising the cerium oxide nanoparticle according to any one of (1) to (8).
(10) An oxidant comprising the cerium oxide nanoparticle according to any one of (1) to (8) or the dispersion solution according to (9).
(11) An antivirus agent comprising the cerium oxide nanoparticle according to any one of (1) to (8) or the dispersion solution according to (9).
(12) An antibacterial agent comprising the cerium oxide nanoparticle according to any one of (1) to (8) or the dispersion solution according to (9).

### Advantageous Effects of Invention

The cerium oxide nanoparticle or the dispersion solution containing this nanoparticle according to the present invention can oxidatively decompose an organic substance and a harmful substance with a higher yield than conventional cerium oxide nanoparticles. The cerium oxide nanoparticle or the dispersion solution containing this nanoparticle according to the present invention may be used as a high-performance antivirus agent that can inactivate various viruses and as an antibacterial agent.

### Brief Description of Drawings

FIG. 1 is the Ce L3 edge XANES spectra of the cerium oxide nanoparticles prepared in Example 1 and Comparative Example 2, as measured in Example 18.
FIG. 2 is the Ce L3 edge XANES spectra of the cerium oxide nanoparticles prepared in Example 2 and Comparative Example 5, as measured in Example 18.
FIG. 3 is the Ce L3 edge XANES spectra of the cerium oxide nanoparticles prepared in Example 12 and Comparative Example 2, as measured in Example 18.
FIG. 4 is the Ce L3 edge XANES spectra of the cerium oxide crystal, cerium (III) carbonate, cerium (III) nitrate, and ammonium cerium (IV) nitrate, as measured in Reference Example 1.

### Description of Embodiments

The cerium oxide nanoparticle according to the present invention is sometimes described, in this specification, simply as the nanoparticle according to the present invention; and also, the dispersion solution containing the cerium oxide nanoparticle according to the present invention is sometimes described, in this specification, simply as the dispersion solution according to the present invention.

The cerium oxide nanoparticle according to the present invention is produced by adding an oxidant to a solution containing a boron compound represented by the following general formula (I) and a cerium (III) ion. Synthesis of the cerium oxide nanoparticle according to the present invention is carried out using a water-soluble salt of cerium as one raw material in water or in a solvent that is compatible with water. From the viewpoints of acquiring moderate hydrophilicity and stable dispersion of the nanoparticle by complexation to a hydroxyl group of a metal oxide, an embodiment is used in which a boron compound having the structure represented by the general formula (I) is used as a stabilizing agent.

BRₙ(OR')₃₋ₙ (I)

In formula (I), n represents an integer of 0 to 2, R represents any of an alkyl group having 1 to 4 carbon atoms, a phenyl group, and a tolyl group, and R' represents any of a hydrogen atom, an alkyl group having 1 to 4 carbon atoms, a phenyl group, and a tolyl group. The plurality of Rs or of R's present may be the same or different from each other.

More preferable embodiments of the boron compound to be used in the present invention include boric acid (in the general formula (I), n = 0, R = H, R' = H), a boric acid ester (in the general formula (I), n = 0, R = H, R' = alkyl group or the like), a boronic acid (in the general formula (I), n = 1, R = alkyl group or the like, R'=H), a boronic acid ester (in the general formula (I), n = 1, R = alkyl group or the like, R' = alkyl group or the like), a borinic acid (in the general formula (I), n = 2, R = alkyl group or the like, R' = H), a borinic acid ester (in the general formula (I), n = 2, R = alkyl group or the like, R' = alkyl group or the like), and a borate salt. In the present invention, the borate salt is a generic term that includes borate salts of metaborate and of polyborate that are formed by dehydration-condensation of boric acid or a borate salt. These borate salts have the structure of boric acid described in the general formula (I) in solution, because in an aqueous solution they are in the equilibrium state between boric acid and tetrahydroxyboric acid. The counter ion of boric acid in the borate salt can be any ion including a lithium ion, a sodium ion, a potassium ion, and an ammonium ion.

Illustrative examples of the boron compound include boric acid; boric acid esters such as trimethyl borate, triethyl borate, tripropyl borate, triisopropyl borate, tributyl borate, and triisobutyl borate; and boronic acids such as methylboronic acid, ethylboronic acid, propylboronic acid, isopropylboronic acid, butylboronic acid, isobutylboronic acid, and phenylboronic acid. Illustrative examples of the borate salt include lithium, sodium, potassium, and ammonium salts of boric acid, metaboric acid, diboric acid, metaboric acid, tetraboric acid, pentaboric acid, hexaboric acid, and octaboric acid.

It is preferable that the cerium oxide nanoparticle according to the present invention contain 0.001 moles or more to 10 moles or less of boron relative to 1 mole of the cerium element. More preferably, the boron content is in the range of 0.001 to 1 moles.

In the present invention, the cerium oxide nanoparticle is composed of a mixture of Ce₂O₃ and CeO₂. The cerium oxide can include the forms of a hydroxide and an oxyhydroxide, in addition to the above oxide forms. The ratio of Ce₂O₃ to CeO₂ can be calculated as the ratio of cerium (III) to cerium (IV) by an X-ray photoelectron spectroscopy (XPS) or other methods.

The cerium oxide nanoparticle according to the present invention can further contain a transition metal belonging to the groups of 3 to 12 in the periodic table. These metals are expected to improve the performance by forming a lattice defect when doped into the cerium oxide nanoparticle by taking a valence of 2+ to 3+, or by causing valence change in the cerium oxide due to the valence changes such as O and 1+, 1+ and 2+, and 2+ and 3+ in accordance with the redox potential thereof.

These transition metals are preferably the transition metals belonging to the groups of 4 to 6 in the periodic table from the viewpoints of being easily doped into the cerium oxide nanoparticle and of improving the antibacterial and antivirus effects, while the transition metals are more preferably Ti, Mn, Fe, Co, Ni, Cu, Zn, Zr, and Ag.

These transition metals can be added during synthesis as organic acid salts such as a carboxylate salt and a sulfonate salt; a phosphate salt; phosphorous oxoacid salts such as a phosphonate salt; inorganic acid salts such as a nitrate salt, a sulfate salt, and a carbonate salt; and salts such as a halide salt and a hydroxide salt. It is preferable that these be soluble in a solvent for the synthesis.

The dispersion solution containing the cerium oxide nanoparticle according to the present invention can be produced by a production method in which the oxidant is added to a solution containing the boron compound and the cerium (III) ion. Hereinafter, the method for producing the cerium oxide nanoparticle according to the present invention will be described.

The first step is to obtain a solution containing the boron compound and the cerium(III) ion. The solution of the boron compound to be used at this step may be prepared by dissolving the boron compound into an arbitrary solvent. The solvent is preferably water or a solvent that is compatible with water. Specifically, illustrative examples of the water-compatible solvent include methanol, ethanol, propanol, isopropanol (2-propanol), butanol, tert-butanol, tetrahydrofuran, acetone, dimethylformamide (DMF), dimethyl sulfoxide (DMSO), glycerol, ethyleneglycol, and oligoethyleneglycol. In the case when the boron compound contains a substituent having 3 or less carbon atoms, the boron compound is preferably dissolved in water; on the other hand, in the case when the boron compound contains a substituent having 4 or more carbon atoms, the boron compound is preferably dissolved in an aqueous solution of 50% ethyleneglycol. When the boron compound is not readily dissolved in a solvent, it may be dissolved by heating or by an ultrasonic treatment.

It suffices if the amount of the boron compound based on the cerium (III) ion is in the range of 0.1 to 1000 mole equivalents, preferably 1 to 200 mole equivalents, more preferably 5 to 200 mole equivalents, while still more preferably 10 to 100 mole equivalents.

In order to obtain the solution containing the boron compound and the cerium (III) ion, a solution of the boron compound and a solution of the cerium (III) ion may be separately prepared, which is then followed by mixing these solutions, or when the solvent of the solution of the boron compound is water or the water-compatible solvent, the cerium (III) salt may be added into the solution of the boron compound, which is then followed by mixing them.

The solution containing the cerium (III) ion may be prepared by dissolving the cerium (III) salt into an arbitrary solvent. Examples of the cerium (III) salt include cerium (III) nitrate hexahydrate.

The amount of the cerium (III) salt to be mixed with the solution of the boron compound can be such that the final concentration of the reaction solution may become in the range of 0.01 to 10% by mass. The resulting mixture solution is preferably mixed for 5 minutes or longer until the solution becomes homogeneous.

At the first step, the solution containing the boron compound and the cerium (III) ion is preferably free from a trivalent or higher carboxylic acid, such as those compounds describe below. Even when such compounds are included, the amount thereof relative to the cerium (III) ion is preferably 0.1 or less by equivalent, while more preferably 0.01 or less by equivalent. Specifically, illustrative examples of the trivalent or higher carboxylic acid include nitrilotriacetic acid (NTA), ethylenediaminetetraacetic acid (EDTA), ethylenediaminedisuccinic acid (EDDS), glycol ether diaminetetraacetic acid (EGTA), diethylenetriamino pentaacetic acid (DTPA), citric acid, hydroxyethyl ethylenediaminetetraacetic acid (HEDTA), and a polyacrylic acid and/or the salt thereof.

When doping the cerium oxide nanoparticle with a metal, a transition metal may be further added at the first step. The transition metal may be added as a solid metal salt directly to the solution containing the boron compound and the cerium (III) ion or the cerium(III) salt, or the solution prepared by dissolving the metal salt in an arbitrary solvent may be added to the solution containing the boron compound and the cerium (III) ion or the cerium (III) salt.

The amount of the transition metal relative to one mole of the cerium (III) ion is preferably in the range of 0.0001 to 0.3 by moles. The amount is more preferably in the range of 0.001 to 0.2 by moles. The amount of the transition metal does not include the amount of an element other than the transition metal contained in the transition metal salt.

At the second step, an oxidant is added into the mixture solution obtained at the first step. Illustrative examples of the oxidant to be used at the second step include nitric acid, potassium nitrate, hypochlorous acid, chlorous acid, chloric acid, perchloric acid, a halogen, a hydrogen halide, a permanganate salt, chromic acid, dichromic acid, oxalic acid, hydrogen sulfide, sulfur dioxide, sodium thiosulfate, sulfuric acid, and hydrogen peroxide. Among these, hydrogen peroxide is especially preferable. The addition amount of the oxidant relative to the cerium (III) ion as the mole equivalent may be in the range of 0.1 or more to 10 or less by mole equivalent, while preferably in the range of 0.5 or more to 2 or less by mole equivalent.

When the oxidant is added to the solution containing the boron compound and the cerium (III) ion, the cerium (III) ion is oxidized to cerium (IV), thereby starting the reaction to form the cerium oxide particle consisting of a mixture of Ce₂O₃ and CeO₂. During this reaction, the solution is colored to yellow, orange, red, brown, or the like. This coloring takes place because the cerium (III) ion changes to cerium (IV), in which the coloring degree is determined by the ratio of cerium (III) to cerium (IV) that are present on the surface of the cerium oxide nanoparticle. Termination of the reaction can be judged at the time when the color does not change any further.

The formation reaction of the cerium oxide nanoparticle can be carried out at an arbitrary pH, but because the reaction can more readily proceed in a weakly acidic solution to a basic solution, it is preferable to keep the pH of the solution at 5 or higher when adding the oxidant, more preferable to control pH at 6 or higher, while even more preferable to control pH at 7 or higher. Upon controlling the pH, an aqueous sodium hydroxide solution or an aqueous ammonia solution may be used. Because the pH of the solution shifts toward an acidic side as the reaction progresses, the pH of the reaction solution may be maintained at 5 or higher from the time of oxidant addition until the end of the reaction. The reaction is usually completed in about 5 minutes to about 1 hour; then, the dispersion solution containing the cerium oxide nanoparticle according to the present invention can be obtained. For example, when 1 ml of an aqueous solution of 10% by mass of cerium (III) nitrate hexahydrate is added to an aqueous boric acid solution of 284 mg/50 ml whose pH having been adjusted at 8, followed by addition of 1 ml of an aqueous solution of 1.2% by mass of hydrogen peroxide and stirring the resulting mixture at room temperature, the solution turns to an orange color, and the reaction to form the particle is completed in about 10 minutes to obtain the dispersion solution according to the present invention.

The formation reaction of the cerium oxide nanoparticle may be carried out at an arbitrary temperature in the range of 4 to 230°C. When the heating is carried out at 100°C or higher, a hydrothermal treatment may be carried out at the temperature in the range of 100 to 230°C. For cooling, a cool bath such as BBL101 manufactured by Yamato Scientific Co., Ltd. may be used, and for heating, a hot bath such as OHB-1100S manufactured by Tokyo Rikakikai Co., Ltd. may be used. When heating is carried out at the temperature of 100°C or lower, this may be carried out by putting the reaction solution in a glass vessel, and when the hydrothermal treatment is carried out at the temperature of 100°C or higher, the reaction solution may be heated in a pressure-resistant vessel consisting of an inner cylinder vessel made of PTFE and an outer cylinder made of a pressure-resistant stainless steel. The hydrothermal treatment may also be carried out by placing the reaction solution in a medium bottle using a sterilizer such as LSX-500 manufactured by TOMY Industries Co., Ltd.

The pH of the dispersion solution according to the present invention may be controlled after the reaction as well. The pH of the dispersion solution according to the present invention may be in the range of 1 to 10, while preferably in the range of 2 to 8. The pH may be controlled by adding a buffer solution, an acid such as nitric acid, sulfuric acid, or hydrochloric acid, or a base such as sodium hydroxide or potassium hydroxide. The pH control of the dispersion solution may also be carried out after purification of the dispersion solution by means of, among other means, filtration through an ultrafiltration membrane or dialysis through a semipermeable membrane; these will be described below.

The dispersion solution according to the present invention may be obtained by removing the unreacted oxidant, the cerium (III) ion, and the excess boron compound that remain in the dispersion solution after completion of the reaction by filtration using an ultrafiltration membrane or by dialysis using a semi-permeable membrane. Then, the cerium oxide nanoparticle may be isolated from the dispersion solution according to the present invention by the method to be described later.

After the purification, the dispersion solution according to the present invention may be heat-treated at an arbitrary temperature in the range of 30 to 230°C. When the heating is carried out at 100°C or higher, the hydrothermal treatment may be carried out at the temperature in the range of 100 to 230°C. For heating, a hot bath such as OHB-1100S, manufactured by Tokyo Rikakikai Co., Ltd., may be used. When heating is carried out at the temperature of 100°C or lower, the heating may be carried out by putting the post-purification dispersion solution in a glass vessel, and when the hydrothermal treatment is carried out at the temperature of 100°C or higher, the post-purification dispersion solution may be heated in a pressure-resistant vessel consisting of an inner cylinder vessel made of PTFE and an outer cylinder made of a pressure-resistant stainless steel. The hydrothermal treatment may also be carried out by placing the post-purification dispersion solution in a medium bottle using a sterilizer such as LSX-500 manufactured by TOMY Industries Co., Ltd.

The cerium oxide nanoparticle according to the present invention may be isolated by drying the dispersion solution according to the present invention using an evaporator, a freeze-dryer, or the like. The cerium oxide nanoparticle may also be isolated by dropping the dispersion solution according to the present invention onto a base plate such as glass, plastics, or ceramics followed by drying with an air or in a desiccator, or with a dryer. The isolation may also be effected by dropping the dispersion solution according to the present invention onto a heat block followed by heating to volatilize the solvent. Further, the isolation may also be effected by drying the dispersion solution according to the present invention with a spray dryer or the like to volatilize the solvent. The isolation may also be effected by centrifuging the dispersion solution according to the present invention to precipitate the cerium oxide nanoparticle followed by removing the supernatant thereof. Also, the cerium oxide nanoparticle may be isolated on a filtration membrane by filtering the dispersion solution according to the present invention with ultrafiltration or suction filtration to completely remove water. In order to make the drying step in the above operations more efficient, an azeotropic solvent may be added to the dispersion solution according to the present invention, or the solvent of the dispersion solution may be replaced with a solvent having a lower boiling point. In order to make the centrifugation operation more efficient, co-precipitants may be added to the dispersion solution according to the present invention, or a solvent may be added to increase an ionic strength or to reduce the dispersibility of the nanoparticles. Prior to the above operations, the dispersion solution according to the present invention may be fractionated in the size of the nanoparticles by the ultrafiltration membrane or the centrifugation.

The dispersion solution according to the present invention may include an ionic component. Illustrative examples of the ionic component that imparts a buffering property include acetic acid, phthalic acid, succinic acid, carbonic acid, tris(hydroxymethyl)aminomethane (Tris), 2-morpholinoethanesulfonic acid monohydrate (MES), bis(2-hydroxyethyl)iminotris(hydroxymethyl)methane (Bis-Tris), N-(2-acetamido)iminodiacetic acid (ADA), piperazine-1,4-bis(2-ethanesulfonic acid) (PIPES), N-(2-acetamido)-2-aminoethanesulfonic acid (ACES), 2-hydroxy-3-morpholinopropanesulfonic acid (MOPSO), N,N-bis(2-hydroxyethyl)-2-aminoethanesulfonic acid (BES), 3-morpholinopropanesulfonic acid (MOPS), N-tris(hydroxymethyl)methyl-2-aminoethanesulfonic acid (TES), 2-[4-(2-hydroxyethyl)-1-piperazinyl]ethanesulfonic acid (HEPES), 2-hydroxy-N-tris(hydroxymethyl)methyl-3-aminopropanesulfonic (TAPSO), piperazine-1,4-bis(2-hydroxy-3-propanesulfonic acid) (POPSO), 2-hydroxy-3-[4-(2-hydroxyethyl)-1-piperazinyl]propanesulfonic acid (HEPSO), 3-[4-(2-hydroxyethyl)-1-piperazinyl]propanesulfonic acid (HEPPS), (Tricine), N,N-bis(2-hydroxyethyl)glycine (Bicine), and N-tris(hydroxymethyl)methyl-3-aminopropanesulfonic acid (TAPS). As for the component that does not impart a buffering property, sodium chloride and potassium chloride may be mentioned. These ionic components may be added such that the final concentration thereof may fall in the range of 0.1 mM to 1 M. These ionic components may be added to the dispersion solution after completion of the reaction, or added after filtration by an ultrafiltration membrane, or used as a dialysate, or added to the dispersed solution after dialysis. These components may also be added to the dried cerium oxide nanoparticle to make the dispersion solution.

The dispersion solution according to the present invention may be stored as the post-reaction dispersion solution, or as a purified product obtained by filtering the post-reaction dispersion solution through an ultrafiltration membrane, or as a purified product having been dialyzed through a semi-permeable membrane, or as an isolated cerium nanoparticle having been dried using an evaporator, a freeze-drier, or the like. Also, this may be stored as a dispersion solution containing a solvent component such as an azeotropic solvent and the ionic component having been additionally added, or as a dispersant whose pH has been controlled. When stored, it is preferable to be stored under a refrigerated state.

The hydrodynamic diameter of the cerium oxide nanoparticle according to the present invention is calculated as the average particle diameter from the number conversion histogram obtained by measuring the dynamic light scattering of the particles to obtain the autocorrelation function followed by analyzing this with the Marquadt method. Measurement of the dynamic light scattering is conducted by using ELS-Z, manufactured by Otsuka Electronics Co., Ltd. The hydrodynamic diameter of the cerium oxide nanoparticle may be in the range of 1 nm or more to 1000 nm or less, while preferably in the range of 1 nm or more to 200 nm or less.

The hydrodynamic diameter of the cerium oxide nanoparticle according to the present invention may be controlled by the reaction temperature. The reaction temperature may be arbitrary set in the range of about 4°C to about 90°C, in which a particle having a smaller diameter can be obtained at a lower reaction temperature, while a particle having a larger diameter can be obtained at a higher reaction temperature.

The cerium oxide nanoparticle or the dispersion solution thereof according to the present invention may be sterilized prior to the use thereof. Illustrative examples of the sterilization method include passing through a sterilization filter, autoclaving (e.g., at 120°C for 20 minutes), and irradiating a 254-nm UV beam.

In the cerium oxide nanoparticle according to the present invention, the energy states of cerium (III) and cerium (IV) in Ce₂O₃ and CeO₂ may be observed by measurement of the X-ray absorption fine structure (XAFS) spectrum. In the XAFS spectrum, the structure at about 20 eV from the absorption edge is called XANES (X-ray absorption near edge structure), and the extended X-ray absorption fine structure appearing at greater than about 100 eV in a high-energy side from the absorption edge is called EXAFS (extended X-ray absorption fine structure). From XANES, the information relating to the valency and structure of the focused atom can be obtained; from the EXAFS analysis, the information relating to the local structure of the sample, as well as the atom species, valency, and distance around the focused atom can be obtained by Fourier transformation of the actual spectrum (corresponding to FT-EXAFS/dynamic radial distribution function). The energy states of cerium (III) and cerium (IV) in the oxidation reduction reaction of cerium oxide are reflected in the peak positions and peak intensity ratio of the local maximum absorptions in the XANES spectrum.

The cerium oxide nanoparticle according to the present invention has the local maximum absorptions in the range of 5726 eV to 5729 eV and in the range of 5735 eV to 5739 eV in the Ce L3 edge XANES spectrum obtained by measurement of the X-ray absorption fine structure spectrum. In other words, the cerium oxide nanoparticle according to the present invention contains the boron compound represented by the general formula (I) and has the local maximum absorptions in the range of 5726 eV to 5729 eV and in the range of 5735 eV to 5739 eV in the XANES spectrum. In another embodiment, the cerium oxide nanoparticle according to the present invention is produced by adding an oxidant to the solution containing the boron compound represented by the general formula (I) and the cerium(III) ion, and has the local maximum absorptions in the range of 5726 eV to 5729 eV and in the range of 5735 eV to 5739 eV in the XANES spectrum.

The cerium oxide nanoparticle or the dispersion solution thereof according to the present invention may be used as an oxidant. For example, by utilizing the oxidative action thereof, this may be used as a homogeneous catalyst in an organic synthetic reaction and in a polymer polymerization, as well as in a solution for wet-etching of a semiconductor. By utilizing the oxidative action thereof, this may be used as the solution that substitutes an oxidative enzyme solution. Specifically, this may be used as a substitute of an oxidase or a peroxidase in a detection reaction or in a tissue dying using an antibody-antigen reaction or a nucleic acid hybridization, or in an electrochemical detection reaction by coating this onto an electrode by immobilizing the cerium oxide nanoparticle. In addition to these, by utilizing the oxidative action thereof, this may be used for degradation and removal of a dirt, an odor, an allergen, a bacterium, a fungus, and a mold, as a bleaching agent or as a disinfectant. Specifically, as the bleaching agent, this may be used for cleaning of cloth, tableware, kitchen, toilet, washroom, bathroom, medical equipment, and so forth. The cleaning method includes soaking and washing, spraying, and spraying using a humidifier or a nebulizer. Also, this may be added as the disinfectant to a swimming pool, a bathtub, and a hot spring; and in addition, this may be used as a body soap, a hand cleaning material, a disinfecting medicine, a gargle, a mouth washer, a hand gel, a sanitizing spray, a disinfectant spray, a wet tissue, a sanitizing sheet, and so forth. The cerium oxide nanoparticle according to the present invention may be allowed to remain on the object after cleaning or disinfection as described above so as to retain the effects as a deodorant, an antivirus agent, an antibacterial agent, and an antifungal agent. The performance of this as the oxidant described above may be evaluated by the color fading reaction of an organic dye or the like, which will be described later.

When the cerium oxide nanoparticle or the dispersion solution thereof according to the present invention is used as the oxidant, this may be used in combination with an alcohol, a surfactant, a disinfectant, or a naturally occurring organic substance. Illustrative examples of the alcohol include ethanol and isopropanol (2-propanol); the surfactant include benzalkonium chloride, benzethonium chloride, and alkyl polyaminoethylglycine; the disinfectant include chlorhexidine, acrinol, merbromin, and crystal violet; and the naturally occurring organic substance include a polyphenol, catechin, tannic acid, chitin, chitosan, isothiocyanate, hinokitiol, limonene, polylysine, terpenoid, saponin, flavonoid, and carotene. A plurality of these may be combined when using.

When the cerium oxide nanoparticle or the dispersion solution thereof according to the present invention is used as an oxidant, this may be used in a combination with another publicly known oxidant. Illustrative examples thereof include hypochlorous acid, sodium hypochlorite, povidone-iodine, oxidol, ozonated water, and peracetic acid; and a plurality of these may be combined.

The color fading reaction of an organic dye is also used for evaluation of the photocatalytic performance of titanium oxide, in which the degradation rate of the dye obtained is used as the indicator of the characteristic with regard to the oxidative degradation of an organic substance. Because low-molecular weight substances such as acetaldehyde and ammonia, as well as various harmful substances such as an allergen are organic substances, the titanium oxide is expected to be used, besides the use as an antibacterial agent, for degradation of various harmful substances because of the characteristics thereof as described above. Similarly, the cerium oxide nanoparticle or the dispersion solution thereof according to the present invention may be used, besides the use as an antibacterial agent, for degradation of various harmful substances if the degradation rate of a dye is high.

The degradation rate of a dye is calculated specifically as follows. First, the dispersion solution according to the present invention is mixed with an organic dye such as Acid Red 94 (AR94); then, the resulting mixture solution is allowed to statically leave for a prescribed period. As a control, the AR94 solution not containing the cerium oxide nanoparticle is treated similarly. After the reaction, the absorption spectra of all the solutions are measured. For analysis, the absorbance at 552 nm, the maximum absorption wavelength of AR94, is used. The difference value between the absorbance of the control (I_{c}) and the absorbance of the solution including the cerium oxide nanoparticle (I), i.e., (I_{c}-I), is obtained; then, the ratio to the absorbance of the control (I_{c}), i.e., [(I_{c}-I)/I_{c} × 100], is calculated as the degradation rate.

Also, a preferable embodiment of the dispersion solution according to the present invention is the dispersion solution that contains the boron compound and the cerium oxide nanoparticle having the degradation rate of 25% or more in the degradation reaction of Acid Red 94 at 40°C for 1 hour. When the degradation rate in the degradation reaction of Acid Red 94 at 40°C for 1 hour is 25% or more, this can be used as the oxidant. The degradation rate in the degradation reaction of Acid Red 94 at 40°C for 1 hour is preferably 50% or more, while especially preferably 70% or more.

The cerium oxide nanoparticle or the dispersion solution thereof according to the present invention may be added, as the additive to impart an oxidative performance, to a fiber, a tube, a bead, a rubber, a film, a plastic, or the like during molding of these materials, or may be applied to the surfaces of these materials for odor preventive, anti-allergic, antibacterial, or anti-fungal processing. Illustrative examples of the processed product using the cerium oxide nanoparticle or the dispersion solution thereof according to the present invention include a chrysanthemum-shaped cover of the drain hole in a kitchen sink, a drain plug, a fixing packing material of a window, a fixing packing material of a mirror, a water-proof packing material in a bathroom, in a washing stand, and in a kitchen, an inner packing material of a refrigerator door, a bath mat, an anti-sliding rubber of a washing bowl and a chair, a hose, a shower head, a packing material used in a water purifier, a plastic product of a water purifier, a packing material used in a clothes washing machine, a plastic product used in a clothes washing machine, a mask, a cap for medical use, a shoes cover for medical use, an air conditioner filter, a filter of air cleaning equipment, a filter for a vacuum cleaner, a filter for a ventilation fan, a filter for a vehicle, a filter for an air conditioner, a fin of an air conditioner, plastic parts such as a rover of an air conditioner's blowing outlet and a blowing fan, a fin of a car air conditioner, plastic parts such as a rover of a car air conditioner's blowing outlet and a blowing fan, clothes, bedding clothes, a net in a screen door, a net for a chicken house, a net such as a mosquito net, a wall paper, a window, a blind, an interior material of a building such as a hospital, an interior material of a train and a car, a seat for a vehicle, a blind, a chair, a sofa, virus-treating equipment, and a construction material for a door, a ceiling, a floor, and a window. Thus, products that are processed using the cerium oxide nanoparticle or the dispersion solution thereof according to the present invention can be used in various fields as hygienic materials.

The cerium oxide nanoparticle or the dispersion solution thereof according to the present invention may be used as an antivirus agent. In the method to evaluate the performance as the antivirus agent, the cerium oxide nanoparticle or the dispersion solution thereof according to the present invention is caused to contact or to be mixed with a virus; then, the amount of the virus is quantified. Illustrative examples of the quantifying method of the virus include: the method in which the virus antigen amount is measured by the ELISA method; the method in which the virus nucleic acid is quantified by PCR; the method in which an infectivity titer is measured by the plaque assay method; and the method in which an infectivity titer is measured by the 50% infectious dose assay method. In the present invention, the antivirus performance is measured preferably by the method in which the infectivity titer is measured by the plaque assay method or by the 50% infectious dose assay method. The unit of the virus infectivity titer in the 50% infectious dose assay method is expressed by: TCID₅₀ (tissue culture infectious dose 50) when the object of the test is a cultured cell; EID₅₀ (egg infectious dose 50) when an embryonated egg is used; and LD₅₀ (lethal dose 50) in an animal. In the 50% infectious dose assay method, illustrative examples of the method to calculate the infectivity titer from the obtained data include the Reed-Muench method, the Behrens-Kaeber method, and the Spearman-Karber method. In the present invention, it is preferable to use the Reed-Muench method. With regard to the judgement standard of the antivirus performance, in general, when the logarithmic reduction value of the infectivity titer relative to the infectivity titer before action of the cerium oxide nanoparticle according to the present invention, or to the control not containing the cerium oxide nanoparticle according to the present invention is 2.0 or more, this is judged to be effective in the antivirus performance.

In another preferable embodiment of the dispersion solution containing the cerium oxide nanoparticle according to the present invention, this includes the boron compound and the cerium oxide nanoparticle, and the logarithmic reduction value in the virus infection titer TCID₅₀ in the 50% infectious dose assay method in the virus inactivation test for the cell culture is 2.0 or more as compare with the infection titer before the action of the cerium oxide nanoparticle according to the present invention or compared with the control not containing the nanoparticle according to the present invention. When the logarithmic reduction value in the virus infection titer TCID₅₀ in the virus inactivation test is 2.0 or more, this can be used as an antivirus agent. The logarithmic reduction value in the virus infection titer is preferably 2.5 or more, while especially preferably 3.0 or more.

Illustrative examples of the virus that can be inactivated by the cerium oxide nanoparticle or the dispersion solution thereof according to the present invention include rhino virus, polio virus, foot and mouth disease virus, rotavirus, norovirus, enterovirus, hepatovirus, astrovirus, sapovirus, hepatitis E virus, influenza A virus, influenza B virus, influenza C virus, parainfluenza virus, mumps virus (epidemic parotitis), measles virus, human metapneumovirus, RS virus, nipah virus, hendra virus, yellow fever virus, dengue virus, Japanese B encephalitis virus, West Nile encephalitis virus, hepatitis B virus, hepatitis C virus, eastern equine encephalitis virus, western equine encephalitis virus, O'nyong'nyong virus, rubella virus, Lassa virus, Junin virus, Machupo virus, Guanarito virus, Sabia virus, Crimean-Congo hemorrhagic fever virus, sandfly virus, hantavirus, Sin Nombre virus, rabies virus, Ebola virus, Marburg virus, bat-borne lyssavirus, human T-cell leukemia virus, human immunodeficiency virus, human corona virus, SARS corona virus, SARS corona virus 2, human parvovirus, polyoma virus, human papilloma virus, adenovirus, herpes virus, varicella zoster virus, EB virus, cytomegalovirus, smallpox virus, monkeypox virus, cowpox virus, Molluscipoxvirus, and parapox virus.

When used as the antivirus agent, the cerium oxide nanoparticle or the dispersion solution thereof according to the present invention may be kneaded as an additive into a textile, a tube, a bead, a rubber, a film, a plastic, or the like, or may be applied onto the surfaces of these materials. Illustrative examples thereof include a mask, a medical cap, a medical shoe cover, a filter for an air conditioner, a filter for an air cleaner, a filter for a vacuum cleaner, a filter for a ventilation fan, a filter for a vehicle, a filter for an air conditioner, a fin of an air conditioner, plastic parts such as a rover of an air conditioner's blowing outlet and a blowing fan, a fin of a car air conditioner, plastic parts such as a rover of a car air conditioner's blowing outlet and a blowing fan, a cloth, a bedding cloth, a net in a screen door, a net for a chicken house, nets such as a mosquito net, a wall paper and a window, a blind, an interior material of a building such as a hospital, an interior material of a train and a car, a seat for a vehicle, a blind, a chair, a sofa, virus-treating equipment, and a construction material in various fields such as a door, a ceiling, a floor, and window.

The cerium oxide nanoparticle or the dispersion solution thereof according to the present invention may be used as an antibacterial agent.

Examples of the method to evaluate the performance as the antibacterial agent include EN1040:2005, which is the European Norm (EN) Test Method. In this test method, a bacteria solution is added to the test solution containing the active ingredient of the antibacterial agent; and then, the number of bacteria is measured after a certain time. The bacteria solution contains 0.85% NaCl and 0.1% tryptone as medium components and is mixed such that the volume ratio, test solution:bacteria solution, becomes 9:1. With regard to the judgement standard of the antibacterial activity, in general, when the logarithmic reduction value of the number of bacteria relative to the number of bacteria before action of the cerium oxide nanoparticle according to the present invention, or to the control not containing the cerium oxide nanoparticle according to the present invention is 2.0 or more, this is judged to be effective in the antibacterial activity. Illustrative examples of the method for quantifying the number of bacteria include the method in which the amount of bacteria is measured by measuring turbidity (OD600), the method in which the amount of bacteria is measured by colony formation, and the method in which the nucleic acid of the bacteria is measured by PCR. In the present invention, the antibacterial performance is measured preferably by the method in which an infectivity titer is measured by the turbidity or by the colony formation.

In another preferable embodiment of the dispersion solution containing the cerium oxide nanoparticle according to the present invention, this includes the boron compound and the cerium oxide nanoparticle, and the logarithmic reduction value of the number of bacteria is 2.0 or more as compared to the infection titer before action of the cerium oxide nanoparticle according to the present invention or to the control not containing the nanoparticle according to the present invention. When the logarithmic reduction value of the number of bacteria in the antibacterial test is 2.0 or more, this can be used as the antibacterial agent. The logarithmic reduction value of the number of bacteria is preferably 2.5 or more, while especially preferably 3.0 or more.

The target microorganisms for which the cerium oxide nanoparticle or the dispersion solution thereof according to the present invention exhibits the antibacterial activity include the following. As for the bacteria, a gram-positive bacterium and a gram-negative bacterium may be mentioned. Illustrative examples of the gram-negative bacterium include bacteria belonging to the genus Escherichia coli such as Escherichia coli, bacteria belonging to the genus Salmonella such as Salmonella, bacteria belonging to the genus Pseudomonas such as Pseudomonas aeruginosa, bacteria belonging to the genus Shigella such as Shigella, bacteria belonging to the genus Klebsiella such as Klebsiella pneumoniae, and bacteria belonging to the genus Legionella such as Legionella pneumophila. Illustrative examples of the gram-positive bacterium include bacteria belonging to the genus Staphylococcus such as Staphylococcus, bacteria belonging to the genus Bacillus such as Bacillus subtilis, and bacteria belonging to the genus Mycobacterium such as Mycobacterium tuberculosis. As for the Eumycetes, fungus and yeast may be mentioned. Illustrative examples of the fungus include filamentous fungi belonging to the genus Aspergillus such as black mold, filamentous fungi belonging to the genus Penicillium such as blue mold, filamentous fungi belonging to the genus Cladosporium such as black mold, filamentous fungi belonging to the genus Alternaria such as sooty mold, filamentous fungi belonging to the genus Trichoderma such as aardvark, and filamentous fungi belonging to the genus Ketomium such as Ketamakabi. Illustrative examples of the yeast include yeasts belonging to the genus Saccharomyces such as baker's yeast and brewer's yeast, and yeasts belonging to the genus Candida such as Candida albicans.

The cerium oxide nanoparticles or the dispersion solution thereof according to the present invention may be added to a disinfectant so as to impart the antivirus action or the antibacterial action to this disinfectant. The disinfectant containing an active ingredient may be the disinfectant added with the cerium oxide nanoparticles or the dispersion solution thereof according to the present invention as described above, in which illustrative examples of the active ingredient include a chlorine-based, an iodine-based, a peroxide-based, an aldehyde-based, a phenol-based, a biguanide-based, a mercury-based, an alcohol-based, an anionic surfactant-based, a cationic surfactant-based, an amphoteric surfactant-based, a nonionic surfactant-based, and a naturally occurring substance-based disinfectant ingredients.

In the case of the disinfectant in a liquid state, the concentration of the cerium oxide nanoparticle according to the present invention may be arbitrarily set in the range of 0.0001% to 10% by mass.

Illustrative examples of the chlorine-based disinfectant ingredient include sodium hypochlorite, chlorine, and chlorinated isocyanuric acid.

Illustrative examples of the iodine-based disinfectant ingredient include iodine, povidone iodide, nonoxynol iodide, and phenoxy iodine.

Illustrative examples of the peroxide-based disinfectant ingredient include hydrogen peroxide, potassium permanganate, peracetic acid, organic peracetic acid, sodium percarbonate, sodium perborate, and ozone.

Illustrative examples of the aldehyde-based disinfectant ingredient include glutaraldehyde, phtharal, and formaldehyde.

Illustrative examples of the phenol-based disinfectant ingredient include isopropyl methylphenol, thymol, eugenol, triclosan, cresol, phenol, chlorocresol, para-chloro-meta-cresol, para-chloro-meta-xylenol, orthophenylphenol, an alkyl para-oxybenzoate ester, resorcin, hexachlorophene, and salicylic acid or the salts thereof.

Illustrative examples of the biguanide-based disinfectant ingredient include chlorhexidine, chlorhexidine gluconate, and chlorhexidine hydrochloride.

Illustrative examples of the mercury-based disinfectant ingredient include mercurochrome, mercuric chloride, and thimerosal.

Illustrative examples of the alcohol-based disinfectant ingredient include ethanol and isopropanol (2-propanol). In this case, the concentration of the alcohol-based disinfectant ingredient is preferably in the range of 30 to 80% by mass.

Illustrative examples of the anionic surfactant-based disinfectant ingredient include an alkylbenzene sulfonate, a fatty acid salt, a higher alcohol sulfate salt, a polyoxyethylene alkyl ether sulfate salt, an α-sulfo fatty acid ester, an α-olefin sulfonate salt, a monoalkyl phosphate ester, and an alkane sulfonate salt.

Illustrative examples of the cationic surfactant-based disinfectant ingredient include an alkyl trimethylammonium salt, a dialkyl dimethylammonium salt, an alkyl dimethyl benzylammonium salt, a polyhexamethylene biguanide, and a benzethonium chloride.

Illustrative examples of the amphoteric surfactant-based disinfectant ingredient include an alkylamino fatty acid salt, an alkyl betaine, and an alkylamine oxide.

Illustrative examples of the nonionic surfactant-based disinfectant ingredient include a polyoxyethylene alkyl ether, a polyoxyethylene-polyoxypropylene alkyl ether, a polyoxyethylene-polyoxybutylene alkyl ether, an alkylamine ethoxylate, an alkylamine alkoxylate, a polyoxyethylene-polyoxypropylene block copolymer, a polyoxyethylene-polyoxypropylene block copolymer (reverse type), an ethylene oxide/propylene oxide adduct of a polyhydric alcohol, an alkyl glucoside, and a fatty acid alkanolamide.

Illustrative examples of the naturally occurring substance-based disinfectant ingredient include: plant-based agents such as hinokitiol, anethole, anise oil, borneol, camphor, carvone, cassia oil, acacia oil, cineole, citral, citronellal, eugenol, pinene, geraniol, lemon oil, liolol, menthol, orange oil, safrole, thymol, and polyphenols (flavanols, gallotannins, ellagitannins, and phlorotannins); animal-based agents such as calcined shell powder obtained by calcination of scallop and oyster shells, such as chitin and chitosan made from crustacean shells; microorganism-based agents such as polylysine; and enzyme-based agents such as lysozyme. An antibacterial peptide produced by an organism to defend itself against an external microorganism may also be used; illustrative examples thereof include histatin, defensin, lactoferrin, lactoferricin, which is a degradation product of lactoferrin, magainin, cecropin, and melititin.

A plant extract may also be used as the naturally occurring substance-based disinfectant ingredient. Specific examples thereof include extract of plants such as grapefruit seed; Chenopodiaceae such as Hahakigi; Iridaceae such as blackberry lily; Hypericaceae such as hypericum perforatum; Bursiaceae such as olibanum and Gilead balsam; Campanulaceae such as Adenophora triphylla; Asteraceae such as Echinacea, Chamomile, Burdock, Solidago canadensis, and Atractylodes lancea; Ranunculaceae such as Coptis rhizome; Caprifoliaceae such as Japanese honeysuckle; Lauraceae such as Laurus nobilis; Moraceae such as hop; Labiatae such as Scutellaria baicalensis, oregano, Schizonepeta tenuifolia, Sage, Thyme, seiyouyamahakka, Mosla japonica, Lavender, and Rosemary; Zingiberaceae such as Hedychium and Ginger; Caprifoliaceae such as Sambucus nigra; Taxodiaceae such as cedar; Apiaceae such as Angelica dahurica and Saposhnikovia divaricata; Polygonaceae such as Polygonum aviculare; Ericaceae such as bearberry leaf; Saururaceae such as Houttuynia cordata; Zygophyllaceae such as caltrop; Vitaceae such as Cayratia japonica; Myrtaceae such as allspice, tea tree, eucalyptus, and Clove; Fabaceae such as Maackia amurensis, Styphnolobium japonicum, Sophora flavescens, adenanthera pavonina, and Senna siamea; Hamamelidaceae such as Liquidambar formosana; Rutaceae such as Phellodendron amurense and Citrus unshiu; Boraginaceae such as comfrey; Berberaceae such as barberry and Nandina domestica; Magnoliaceae such as Magnolia obovata; Rosaceae such as great burnet and rose; Loranthaceae such as Mistletoe; Liliaceae such as nemarrhena asphodeloides, Aspidistra elatior, and Glycyrrhiza; Gentianaceae such as Gentiana macrophylla Pall; Poaceae such as moso bamboo; and Fucaceae such as Ascophyllum nodsum.

Ultrafine bubbles include bubbles having a particle diameter of 500 nm or less that contain inside thereof one or two or more gases selected from air, oxygen, hydrogen, nitrogen, carbon dioxide, argon, neon, xenon, fluorinated gas, ozone, and inert gas. The ultrafine bubbles are also called nanobubbles. The concentration thereof may be 100,000 cells/ml or more.

The disinfectant containing the cerium oxide nanoparticle or the dispersion solution thereof according to the present invention may contain, in addition to the disinfectant ingredients listed above, an arbitrary ingredient as appropriate in accordance with the formulation thereof. Specifically, the examples thereof include a solvent, a wetting agent, a thickener, an antioxidant, a pH adjuster, an amino acid, a preservative, a sweetening agent, a fragrance, a surfactant, a colorant, a disinfectant aid, a chelating agent, a UV absorber, an antifoaming agent, an enzyme, and a formulation stabilizer.

The disinfectant added with the cerium oxide nanoparticle or the dispersion solution thereof according to the present invention may be provided in various forms, such as liquid, gel, and powder. The liquid disinfectant may be provided in the form of a lotion, a spray, or the like, and may be filled into a bottle having a measuring cap, a trigger-type spray container, a squeeze-type or a dispenser-type pump spray container, or the like; this may be used by scattering or spraying. The liquid disinfectant may be provided as wet sheets by impregnating into sheet paper or cloth, and then filling this into a bottle, a bucket, or the like.

The cerium oxide nanoparticle according to the present invention may be used for antibacterial processing by adding, as the additive, to a fiber, a tube, a bead, a rubber, a film, a plastic, or the like during molding thereof, or by applying to the surfaces of these materials. Illustrative examples of the product that can be antibacterial-processed by using the cerium oxide nanoparticle or the dispersion solution thereof according to the present invention include a chrysanthemum-shaped cover of the drain hole in a kitchen sink, a drain plug, a fixing packing material of a window, a fixing packing material of a mirror, a water-proof packing material in a bathroom, in a washing stand, and in a kitchen, an inner packing material of a refrigerator door, a bath mat, an anti-sliding rubber of a washing bowl and a chair, a hose, a shower head, a packing material used in a water purifier, a plastic product of a water purifier, a packing material used in a clothes washing machine, a plastic product used in a clothes washing machine, a mask, a cap for medical use, a shoes cover for medical use, an air conditioner filter, a filter of air cleaning equipment, a filter for a vacuum cleaner, a filter for a ventilation fan, a filter for a vehicle, a filter for an air conditioner, a fin of an air conditioner, plastic parts such as a rover of an air conditioner's blowing outlet and a blowing fan, a fin of a car air conditioner, plastic parts such as a rover of a car air conditioner's blowing outlet and a blowing fan, clothes, bedding clothes, a net in a screen door, a net for a chicken house, a net such as a mosquito net, a wall paper and a window, a blind, an interior material of a building such as a hospital, an interior material of a train and a car, a seat for a vehicle, a blind, a chair, a sofa, virus-treating equipment, and a construction material for a door, a ceiling, a floor, and a window. Thus, products that are processed using the dispersion solution of the cerium oxide nanoparticle according to the present invention can be used in various fields as the hygienic material.

By adding the cerium oxide nanoparticle or the dispersion solution thereof according to the present invention to paint, the antivirus activity can be imparted to this paint. A resin emulsion composition may be contained in the paint for the purpose to immobilize the cerium oxide nanoparticle according to the present invention into the coated film.

Illustrative examples of the resin emulsion composition include a vinyl acetate resin emulsion, a vinyl chloride resin emulsion, an epoxy resin emulsion, an acrylic resin emulsion, a urethane resin emulsion, an acrylic silicone resin emulsion, a fluorinated resin emulsion, or a synthetic resin emulsion consisting of a composite system of these resin components. The mass ratio of the cerium oxide nanoparticle according to the present invention added to the paint to the solid component in the resin emulsion may be arbitrarily set in the range of 0.01:99.99 to 99.99:0.01.

The ethylene-vinyl acetate copolymer resin emulsion is the copolymer of ethylene and vinyl acetate monomer, in which this may be further copolymerized with a monomer having a functional group such as an amino group, a secondary amino group, a tertiary amino group, a quaternary amino group, a carboxyl group, an epoxy group, a sulfonic acid group, a hydroxyl group, a methylol group, or an alkoxy acid group.

The vinyl chloride copolymer resin emulsion is a polymer of vinyl chloride, in which this may be further copolymerized with a vinyl monomer having a functional group such as an amino group, a secondary amino group, a tertiary amino group, a quaternary amino group, a carboxyl group, an epoxy groups, a sulfonic acid group, a hydroxyl group, a methylol group, and an alkoxy acid group.

Illustrative examples of the monomer that can be used for preparation of the acrylic resin emulsion include: (meth)acrylate ester-type monomers such as methyl (meth)acrylate, ethyl (meth)acrylate, propyl (meth)acrylate, butyl (meth)acrylate, hexyl (meth)acrylate, heptyl (meth)acrylate, octyl (meth)acrylate, octadecyl (meth)acrylate, 2-ethylhexyl (meth)acrylate, cyclohexyl (meth)acrylate, nonyl (meth)acrylate, dodecyl (meth)acrylate, stearyl (meth)acrylate, isobornyl (meth)acrylate, dicyclopentanyl (meth)acrylate, and benzyl (meth)acrylate; unsaturated bond-containing monomers having a carboxyl group such as acrylic acid, methacrylic acid, β-carboxyethyl (meth)acrylate, 2-(meth)acryloyl propionic acid, crotonic acid, itaconic acid, maleic acid, fumaric acid, itaconic acid half ester, maleic acid half ester, maleic anhydride, and itaconic anhydride; polymerizable monomers containing a glycidyl group such as glycidyl (meth)acrylate and allyl glycidyl ether; polymerizable monomers containing a hydroxy group such as 2-hydroxyethyl (meth)acrylate, 2-hydroxypropyl (meth)acrylate, poly(ethylene glycol) mono(meth)acrylate, and glycerol mono(meth)acrylate; and ethyleneglycol di(meth)acrylate, 1,6-hexanediol di(meth)acrylate, neopentyl glycol di(meth)acrylate, trimethylolpropane tri(meth)acrylate, polyethyleneglycol di(meth)acrylate, polypropyleneglycol di(meth)acrylate), diallyl phthalate, divinyl benzene, and allyl (meth)acrylate.

Illustrative examples of the monomer that can be used for preparation of the urethane resin emulsion include, as the polyisocyanate component: 2,4-tolylene diisocyanate, 2,6-tolylene diisocyanate, m-phenylene diisocyanate, p-phenylene diisocyanate, 4,4'-diphenylmethane diisocyanate, 2,4'-diphenylmethane diisocyanate, 2,2'-diphenylmethane diisocyanate, 3,3'-dimethyl-4,4 biphenylene diisocyanate, 3,3'-dimethoxy-4,4'-biphenylene diisocyanate, 3,3'-dichloro-4,4'-biphenylene diisocyanate, 1,5 naphthalene diisocyanate, 1,5-tetrahydronaphthalene diisocyanate, 1,6-hexamethylene diisocyanate, dodecamethylene diisocyanate, trimethylhexamethylene diisocyanate, 1,3-cyclohexylene diisocyanate, 1,4-cyclohexylene diisocyanate, xylylene diisocyanate, tetramethylxylylene diisocyanate, hydrogenated xylylene diisocyanate, lysine diisocyanate, isophorone diisocyanate, 4,4'-dicyclohexylmethane diisocyanate, and 3,3'-dimethyl-4,4'-dimethylene diisocyanate; and as the diol component, polyester polyol, polyether polyol, polycarbonate polyol, polyacetal polyol, polyacrylate polyol, polyesteramide polyol, polythioether polyol, and polyolefin polyol such as polybutadiene polyol.

Illustrative examples of the silicon-containing acrylic monomer that can be used for preparation of the acrylic silicon resin emulsion include γ-(meth)acryloxypropyl trimethoxysilane, γ-(meth)acryloxypropyl triethoxysilane, γ-(meth)acryloxypropyl methyl dimethoxysilane, and γ-(meth)acryloxypropyl methyl diethoxysilane.

Illustrative examples of the monomer that can be used for preparation of the fluorinated resin emulsion include fluorinated olefins (vinylidene fluoride, trifluoroethylene, chlorotrifluoroethylene, tetrafluoroethylene, pentafluoroethylene, hexafluoropropylene, etc.), and fluorinated (meth)acrylates (trifluoroethyl (meth)acrylate, pentafluoropropyl (meth)acrylate, perfluorocyclohexyl (meth)acrylate, etc.).

The paint containing the cerium oxide nanoparticle or the dispersion solution thereof according to the present invention may optionally contain a pigment, a matting agent, an aggregate, a fiber, a cross-linking agent, a plasticizer, a preservative, an anti-mold agent, an antibacterial agent, an antifoaming agent, a viscosity regulator, a leveling agent, a pigment dispersant, an antisettling agent, an anti-tagging agent, a UV absorber, a light stabilizer, an antioxidant, and an adsorbent. These components may be blended in a paint composition alone or in combination.

The paint added with the cerium oxide nanoparticle or the dispersion solution thereof according to the present invention may be used, for example, for the paint of an interior surface of a building. Illustrative examples of the interior surface include a mortar, a concrete, a gypsum board, a siding board, an extruded board, a slate board, an asbestos cement board, a fiber-mixed cement board, a calcium silicate board, an ALC board, a metal, a wood, a glass, a rubber, a ceramic, a fired tile, a porcelain tile, a plastic, a base material such as a synthetic resin, a cloth, a wallpaper, or a coating film formed on these substrates. This can also be applied to an exterior surface of a building and a structure other than the building.

### [EXAMPLES]

The present invention will be described more specifically by the following Examples.

### <Materials and Methods>

Cerium(III) nitrate hexahydrate, boric acid, sodium tetraborate decahydrate (borax), ethyleneglycol, and an aqueous 30% by mass hydrogen peroxide were purchased from FUJIFILM Wako Pure Chemical Industries Ltd.; Acid Red 94, trimethyl borate, triethyl borate, isopropyl borate, methylboronic acid, ethylboronic acid, phenylboronic acid, EDTA-2Na, and DL-lactic acid were purchased from Tokyo Chemical Industry Co., Ltd. The commercially available cerium oxide dispersion solution (796077) used in Comparative Examples was purchased from Merck KGaA. Amicon Ultra 15 (30 kD) used for purification was purchased from Merck Millipore Corp.

Other reagents were purchased from FUJIFILM Wako Pure Chemical Industries Ltd., Tokyo Chemical Industry Co., Ltd., and Sigma-Aldrich Co. LLC; and these were used as they were without any further purification.

The zeta potential and particle measurement system ELS-Z manufactured by Otsuka Electronics Co., Ltd. was used for measurement of the hydrodynamic diameter of the cerium oxide nanoparticle; SpectraMax iD3 manufactured by Molecular Devices LLC was used for the plate reader in the absorbance measurement.

### (Example 1) Preparation of Dispersion Solution of Cerium Oxide Nanoparticles Using Boric Acid as Stabilizing Agent

After 50 ml of water was added to a round-bottle flask, 284 mg of boric acid was dissolved in it, and then, the pH of the resulting solution was adjusted to 8.0 with sodium hydroxide. To this was added 1 ml of the aqueous solution of 10% by mass of cerium (III) nitrate hexahydrate; and then, they were stirred at room temperature for 10 minutes. Next, 1 ml of the aqueous solution of 1.2% by mass of hydrogen peroxide was added dropwise; and then, they were allowed to react at room temperature for 1 hour. After the reaction, nitric acid was added; and then, the resulting mixture was stirred at room temperature for 2 hours. The reaction solution thus obtained was purified by using a 10 kD ultrafiltration membrane to obtain an orange-colored dispersion solution containing the cerium oxide nanoparticles.

### (Example 2) Preparation of Dispersion Solution of Cerium Oxide Nanoparticles Using Trimethyl Borate as Stabilizing Agent

The reaction was carried out under the same conditions as those of Example 1, except that in place of 284 mg of boric acid, 388 mg of trimethyl borate was used in Example 1, to obtain an orange-colored dispersion solution containing the cerium oxide nanoparticles.

### (Example 3) Preparation of Dispersion Solution of Cerium Oxide Nanoparticles Using Triethyl Borate as Stabilizing Agent

The reaction was carried out under the same conditions as those of Example 1, except that in place of 284 mg of boric acid, 545 mg of triethyl borate was used in Example 1, to obtain an orange-colored dispersion solution containing the cerium oxide nanoparticles.

### (Example 4) Preparation of Dispersion Solution of Cerium Oxide Nanoparticles Using Tri-isopropyl Borate as Stabilizing Agent

The reaction was carried out under the same conditions as those of Example 1, except that in place of 284 mg of boric acid, 702 mg of tri-isopropyl borate was used, and that in place of 50 ml of water, 50 ml of a 50% by volume of aqueous ethyleneglycol solution was used in Example 1, to obtain an orange-colored dispersion solution containing the cerium oxide nanoparticles.

### (Example 5) Preparation of Dispersion Solution of Cerium Oxide Nanoparticles Using Sodium Tetraborate as Stabilizing Agent

The reaction was carried out under the same conditions as those of Example 1, except that in place of 284 mg of boric acid, 1.42 g of sodium tetraborate decahydrate (borax) was used in Example 1, to obtain an orange-colored dispersion solution containing the cerium oxide nanoparticles.

### (Example 6) Preparation of Dispersion Solution of Cerium Oxide Nanoparticles Using Methylboronic Acid as Stabilizing Agent

The reaction was carried out under the same conditions as those of Example 1, except that in place of 284 mg of boric acid, 223 mg of methylboronic acid was used in Example 1, to obtain a pale brown-colored dispersion solution containing the cerium oxide nanoparticles.

### (Example 7) Preparation of Dispersion Solution of Cerium Oxide Nanoparticles Using Ethylboronic Acid as Stabilizing Agent

The reaction was carried out under the same conditions as those of Example 1, except that in place of 284 mg of boric acid, 276 mg of ethylboronic acid was used in Example 1, to obtain a pale brown-colored dispersion solution containing the cerium oxide nanoparticles.

### (Example 8) Preparation of Dispersion Solution of Cerium Oxide Nanoparticles Using Phenylboronic Acid as Stabilizing Agent

The reaction was carried out under the same conditions as in Example 1, except that in place of 284 mg of boric acid, 455 mg of phenylboronic acid was used, and that in place of 50 ml of water, 50 ml of a 50% by volume of aqueous ethyleneglycol solution was used in Example 1, to obtain a brown-colored dispersion solution containing the cerium oxide nanoparticles.

### (Example 9) Preparation of Dispersion Solution of Cerium Oxide Nanoparticles Using Boric Acid (Boric Acid Solution: pH 4.0) as Stabilizing Agent

The reaction was carried out under the same conditions as those of Example 1, except that pH of the boric acid solution was made 4.0 in Example 1, to obtain a yellow-colored dispersion solution containing the cerium oxide nanoparticles.

### (Example 10) Preparation of Dispersion Solution of Cerium Oxide Nanoparticles Using Boric Acid (Boric Acid Solution: pH 5.0) as Stabilizing Agent

The reaction was carried out under the same conditions as those of Example 1, except that pH of the boric acid solution was made 5.0 in Example 1, to obtain an orange-colored dispersion solution containing the cerium oxide nanoparticles.

### (Example 11) Preparation of Dispersion Solution of Cerium Oxide Nanoparticles Using Boric Acid (Boric Acid Solution under heating at 70°C) as Stabilizing Agent

The reaction was carried out under the same conditions as those of Example 1, except that stirring after addition of nitric acid was carried out at 70°C in Example 1, to obtain an orange-colored dispersion solution containing the cerium oxide nanoparticles.

### (Example 12) Preparation of Dispersion Solution of Cerium Oxide Nanoparticles Using Boric Acid (Boric Acid Solution under heating at 90°C) as Stabilizing Agent

The reaction was carried out under the same conditions as those of Example 1, except that stirring after addition of nitric acid was carried out at 90°C in Example 1, to obtain an orange-colored dispersion solution containing the cerium oxide nanoparticles.

### (Comparative Example 1) Preparation of Dispersion Solution of Cerium Oxide Nanoparticles Using Polyacrylic Acid as Stabilizing Agent

With reference to Non Patent Literature 1, in order to compare the oxidation activity, the cerium oxide nanoparticle was prepared using polyacrylic acid as the stabilizing agent. To 50 ml of the aqueous solution of 1% by mass of polyacrylic acid, 1 ml of the aqueous solution of 10% by mass of cerium (III) nitrate hexahydrate was added; then, they were stirred at room temperature for 5 minutes. Next, 1 ml of the aqueous solution of 1.2% by mass of hydrogen peroxide was added; and then, the reaction was carried out by warming at 40°C for 1 hour. The reaction solution thus obtained was purified by using a 30 kD ultrafiltration membrane to obtain a yellow-colored dispersion solution containing the cerium oxide nanoparticles.

### (Comparative Example 2) Preparation of Dispersion Solution Containing Cerium Oxide Nanoparticles with Post-Addition of Boric Acid

Referring to Patent Literature 1 (Japanese Patent Application Laid-open No. 2003-183631), in order to compare the oxidation performance with Example 1, the dispersion solution was prepared by a producing method in which boric acid was post-added to the dispersion solution of the cerium oxide nanoparticles so as to be adsorbed. A commercially available dispersion solution of the cerium oxide nanoparticle (IV) (Merck, 796077) was diluted to 0.2 mg/ml; and then, after 284 mg of boric acid was added to 50 ml of this diluted solution, the resulting mixture was stirred at 60°C for 2 hours. The reaction solution thus obtained was then purified by using a 10 kD ultrafiltration membrane to obtain a brown-colored dispersion solution containing the cerium oxide nanoparticles.

### (Comparative Example 3) Preparation of Dispersion Solution of Cerium Oxide Nanoparticles Using EDTA/Lactic Acid as Stabilizing Agent

With reference to Patent Literature 2 (Japanese Unexamined Patent Application Publication (Translation of PCT Application) No. 2010-502821), in order to compare the oxidation performance with that of Example 1, the dispersion solution of the cerium oxide nanoparticles was prepared using EDTA/lactic acid as the stabilizing agent.

Cerium (III) nitrate hexahydrate (0.8 g), 0.25 g EDTA-2Na, and 0.25 g DL-lactic acid were dissolved in 50 ml water; and then, the pH of the resulting mixture was adjusted to 9.5 with an aqueous 30% ammonia. To this, 640 µl of an aqueous solution of 30% hydrogen peroxide was added dropwise; and then, the resulting mixture was stirred for 1 hour to obtain a brown-colored aqueous solution. The reaction solution thus obtained was then purified by using a 3 kD ultrafiltration membrane to obtain a brown-colored dispersion solution containing the cerium oxide nanoparticles using EDTA/lactic acid as the stabilizing agent.

### (Comparative Example 4) Preparation of Dispersion Solution of Cerium Oxide Nanoparticles with Post-addition of Boric Acid Using EDTA/Lactic Acid as Stabilizing Agent

The dispersion solution containing the cerium oxide nanoparticles with EDTA/lactic acid as the stabilizing agent obtained in Comparative Example 3 was diluted to 0.2 mg/ml; then 284 mg of boric acid was added to 50 ml of this diluted solution. The reaction solution thus obtained was purified by using a 3 kD ultrafiltration membrane to obtain a brown-colored aqueous solution containing the cerium oxide nanoparticles.

### (Comparative Example 5) Preparation of Dispersion Solution Containing Cerium Oxide Nanoparticles with Post-Addition of Triethyl Borate

In Comparative Example 2, the reaction was carried out under the same conditions as those in Comparative Example 2, except that in place of 284 mg of boric acid, 545 mg of triethyl borate was used, to obtain a brown-colored dispersion solution containing the cerium oxide nanoparticles.

### (Example 13) Measurement of Hydrodynamic Diameter of Cerium Oxide Nanoparticle

The hydrodynamic diameter of the cerium oxide nanoparticle prepared in each of Examples 1 to 12 was measured by the dynamic light scattering (DLS) method. Water was used as the solvent for the measurement; the average particle diameter of the hydrodynamic diameter was obtained in terms of number conversion. The obtained values are listed in Table 1.

The average particle diameters were in the range of 3.4 to 71.0 nm, so that all of them were confirmed to be nanoparticles.

**Table 1**

| Dispersion solution | Stabilizing Agent | Solution | Particle diameter [nm] |
|---|---|---|---|
| Example 1 | Boric acid | Orange | 4.8 ± 0.7 |
| Example 2 | Trimethyl borate | Orange | 15.6 ± 6.0 |
| Example 3 | Triethyl borate | Orange | 7.2 ± 1.0 |
| Example 4 | Isopropyl borate | Orange | 14.1 ± 3.4 |
| Example 5 | Sodium tetraborate | Orange | 9.7 ± 2.1 |
| Example 6 | Methylboronic acid | Pale brown | 71.0 ± 12.0 |
| Example 7 | Ethylboronic acid | Pale brown | 32.6 ± 6.2 |
| Example 8 | Phenylboronic acid | Brown | 13.0 ± 1.0 |
| Example 9 | Boric acid (pH 4.0) | Yellow | 3.4 ± 0.7 |
| Example 10 | Boric acid (pH 5.0) | Orange | 4.7 ± 1.1 |
| Example 11 | Boric acid (Heating at 70°C) | Orange | 15.3 ± 3.6 |
| Example 12 | Boric acid (Heating at 90°C) | Orange | 54.3 ± 28.2 |

### (Example 14) Measurement of Oxidative Performance by Dye Degradation Test

Into 60 µl of each of the dispersion solutions of the cerium oxide nanoparticles prepared in Examples 1 to 12 with the concentration thereof having been adjusted to 2 mg/ml were added 60 µl of 0.5 mg/ml Acid Red 94 (AR94) as the sample including the organic substance, and 1.38 ml of distilled water; then, the resulting mixture was allowed to statically leave at 40°C for 1 hour using a heat block to carry out the degradation reaction of the dye. As the control, the AR94 solution not containing the cerium oxide nanoparticles was treated in the same way as above. After the reaction, 100 µl of each of the solutions was taken and diluted with 1.9 ml of distilled water; then, the absorption spectrum thereof was measured. With regard to the sample of the control, there was no change found in the absorption spectrum before and after the heating.

In the analysis, the absorbance at 552 nm, the wavelength at the maximum absorption of AR94, was used. The difference value between the absorbance (I) of the dispersion solution and the absorbance of the control (I_{c}) was calculated; then, the ratio of this difference value to the absorbance of the control (I_{c}) was calculated as the degradation rate. The results are listed in Table 2.

From these results, it was confirmed that the dispersion solutions containing the cerium oxide nanoparticles of Examples 1 to 12 have the oxidative performance capable of decomposing the dye with a high degradation rate.

On the other hand, the oxidative performances of the dispersion solution of the commercially available cerium oxide nanoparticles and of the dispersion solution of the cerium oxide nanoparticle prepared in Comparative Examples 1 to 5 were measured in the same way; here, the degradation of the dye was hardly recognizable.

**Table 2**

| Nanoparticle | Dye degradation yield [%] |
|---|---|
| Example 1 | 91 |
| Example 2 | 94 |
| Example 3 | 89 |
| Example 4 | 93 |
| Example 5 | 94 |
| Example 6 | 48 |
| Example 7 | 32 |
| Example 8 | 27 |
| Example 9 | 35 |
| Example 10 | 62 |
| Example 11 | 95 |
| Example 12 | 93 |
| Commercial product | 10 |
| Comparative Example 1 | 6 |
| Comparative Example 2 | 9 |
| Comparative Example 3 | 3 |
| Comparative Example 4 | 4 |
| Comparative Example 5 | 7 |

### (Example 15) Virus Inactivation Test

This test was carried out at Kitasato Research Center for Environmental Science. Into 0.9 ml of each of the dispersion solutions of the cerium oxide nanoparticles that had been prepared in Examples 1, 11, and 12, the concentration thereof having been adjusted to 5 mg/mL, was added 0.1 ml of the virus solution (feline calicivirus F-9, ATCC, VR-782, substitute of norovirus); and then, the resulting mixture was allowed to be acted for 1 hour. Then, PBS was added as the action-stopping solution, so that the action to the virus was stopped. The infectivity titer was measured with the TCID₅₀ method by using this solution as the original solution of the sample to measure the virus value.

The logarithmic reduction values of the infectivity titer to the infectivity titer before applying the cerium oxide nanoparticles are listed in Table 3. Because the logarithmic reduction values of the cerium oxide nanoparticles of Examples 1, 11, and 12 were 3.7 to 4.7 in these results, it was confirmed that the cerium oxide nanoparticles according to the present invention had a very high antivirus activity because the virus inactivating rates thereof were 99.9% or more.

In order to compare with the dispersion solution of the cerium oxide nanoparticles that was obtained in Example 1, those sterilized through a 0.2-µm sterilization filter, autoclaved (hydrothermal treatment at 120°C for 20 minutes), and by 254 nm UV irradiation were evaluated in the same way as above. It was found that the virus inactivation rate was the same for all sterilization treatments, and thereby it was confirmed that these treatments had a very high antivirus activity.

On the other hand, in the dispersion solution of the cerium oxide nanoparticles prepared in Comparative Example 1, the logarithmic reduction value was -0.5; thus, this failed to confirm the virus inactivating performance.

**Table 3**

| Dispersion solution | | Logarithmic reduction value in the infection titer |
|---|---|---|
| Boric acid | Example 1 | 3.7 |
| Boric acid (Heating at 70°C) | Example 11 | 4.7 |
| Boric acid (Heating at 90°C) | Example 12 | 4.7 |
| Polyacrylic acid | Comparative Example 1 | -0.5 |

### (Example 16) Virus Inactivation Test Against SARS-CoV-2

This test was carried out at Japan Textile Products Quality and Technology Center. Into 0.9 ml of the dispersion solution of the cerium oxide nanoparticles prepared in Example 12, the concentration thereof having been adjusted to 5 mg/ml, was added 0.1 ml of the virus solution (novel coronavirus, Severe Acute Respiratory Syndrome Coronavirus 2 (SARS-CoV-2), NIID isolated strain: JPN/TY/WK-521 (obtained from the National Institute of Infectious Diseases)); and then, the resulting mixture was allowed to be acted for 1 hour. Then, PBS was added as the action-stopping solution, so that the action to the virus was stopped. The infectivity titer was measured with the plaque assay method by using this solution as the original solution of the sample to measure the virus value.

The logarithmic reduction value of the infectivity titer to the infectivity titer before applying the cerium oxide nanoparticles is listed in Table 4. Because the logarithmic reduction value of the cerium oxide nanoparticles of Example 12 was 3.03 or more, it was confirmed that the cerium oxide nanoparticles according to the present invention had the virus inactivating rate against the SARS-CoV-2 was 99.9% or more.

**Table 4**

| Dispersion solution | | logarithmic reduction value in the infection titer |
|---|---|---|
| Boric acid (Heating at 90°C) | Example 12 | Not less than 3.03 |

### (Example 17) Determination of Ce and B Using ICP Emission Spectrometry and ICP-MS

Each of the dispersion solutions of the cerium oxide nanoparticles in Examples 1, 2, and 12 was weighed into a Teflon (registered trademark) container, decomposed by heating with sulfuric acid, nitric acid, and hydrochloric acid, concentrated until generating a sulfuric acid white smoke, and then dissolved in a dilute aqua regia to obtain a solution with a certain volume. Ce in the resulting solution was determined by ICP emission spectrometry and B by ICP mass analysis. The ICP emission spectrometer used was PS3520VDDII (manufactured by Hitachi High-Tech Science Corp.), and the ICP mass analysis instrument used was Agilent 8800 (manufactured by Agilent Technologies Inc.). The obtained values are listed in Table 5.

The amount of boric acid was found to be 0.016 to 0.0541 moles relative to 1 mole of Ce.

**Table 5**

| | Boron content relative to 1 mole Ce [mol] |
|---|---|
| Example 1 | 0.0175 |
| Example 2 | 0.0160 |
| Example 12 | 0.0541 |

### (Example 18) XAFS Analysis of Cerium Oxide Nanoceria

An X-ray was irradiated to the dispersion solution of the cerium oxide nanoparticles according to the present invention that had been prepared in Example 1 and had the concentration thereof adjusted to 10 mg/mL; then, the absorption amount thereof was measured to obtain the X-ray absorption fine structure (XAFS) spectrum. The measurement was carried out at High Energy Accelerator Research Organization Synchrotron Radiation Research Center (Photon Factory) using BL12C and the Si(111) double crystal spectrometer with the absorption edge being the Ce L3 absorption edge by the transmission detection method using the ion chamber detector.

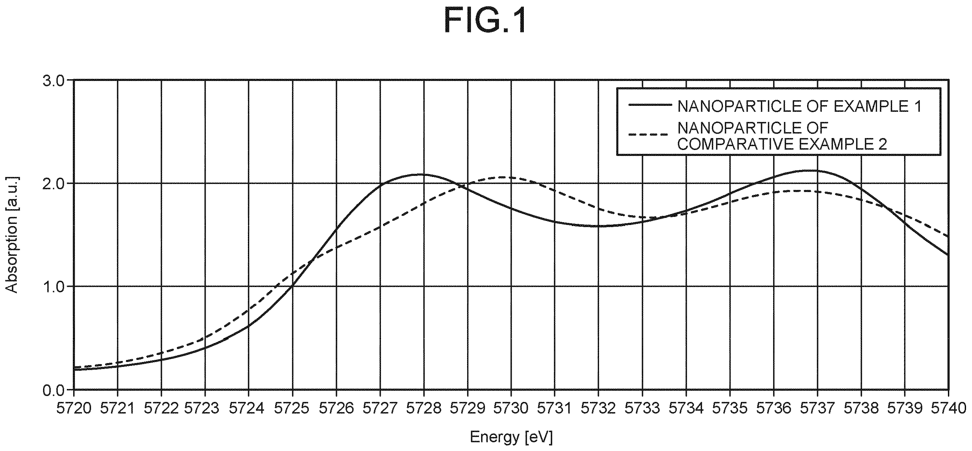

The Ce L3 edge XANES spectrum thereof is exhibited in FIG. 1. In the spectra, the absorption edge (E0) of the spectrum was set at 5724.4 eV, in which the vertical axis is the ratio based on 0 as the average absorption in the range of -150 eV to -30 eV from E0, and 1 as the average absorption in the range of +150 eV to +400 eV from E0.

With regard to the dispersion solution of the cerium oxide nanoparticle prepared in Comparative Example 2, the XAFS observation was also carried out with the same operations under the same conditions as those described above. The Ce L3 edge XANES spectrum thus obtained is exhibited in FIG. 1.

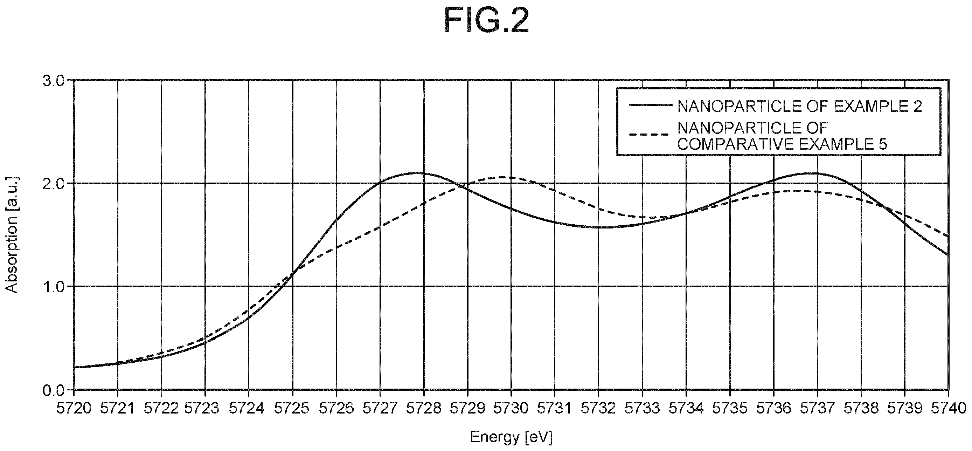

With regard to the dispersion solutions of the cerium oxide nanoparticles prepared in Example 2 and Comparative Example 5, the XAFS observations were also carried out with the same operations under the same conditions as those described above; the Ce L3 edge XANES spectra thus obtained are exhibited in FIG. 2.

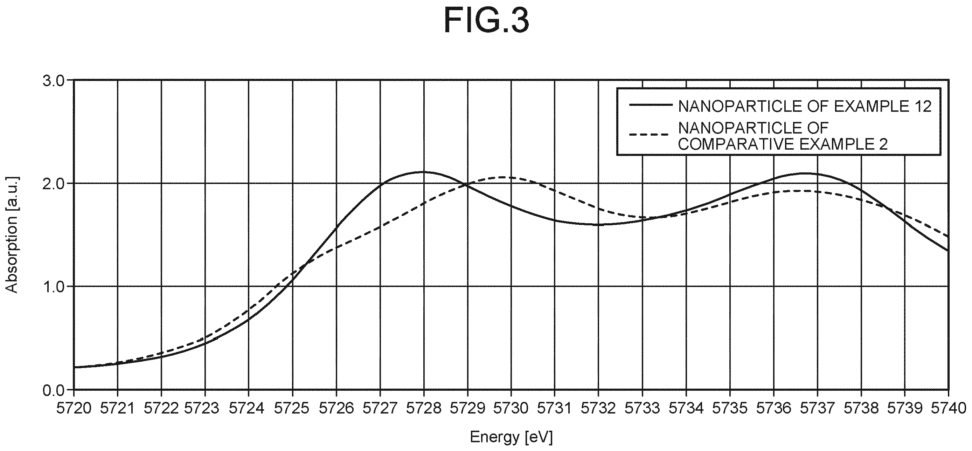

With regard to the dispersion solutions of the cerium oxide nanoparticles prepared in Example 12 and Comparative Example 2, the XAFS observations were also carried out with the same operations under the same conditions as those described above; the Ce L3 edge XANES spectra thus obtained are exhibited in FIG. 3.

These results indicate that the nanoparticles of Example 1 have the local maximum absorptions at 5727.974 eV and 5736.694 eV, those of Example 2 have the local maximum absorptions at 5727.705 eV and 5736.964 eV, and those of Example 12 have the local maximum absorptions at 5728.078 eV and 5736.568 eV; thus, it became clear that these have the local maximum absorptions at 5726 eV to 5729 eV and 5735 eV to 5739 eV.

On the other hand, the cerium oxide nanoparticles of Comparative Example 2 have the local maximum absorptions at 5729.732 eV and 5736.694 eV, and those of Comparative Example 5 have the local maximum absorptions at 5729.810 eV and 5736.568 eV; thus, it was found that these have the local maximum absorptions in the range of 5735 eV to 5739 eV, but do not have the local maximum absorptions in the range of 5726 eV to 5729 eV.

### (Reference Example 1) XAFS Observation

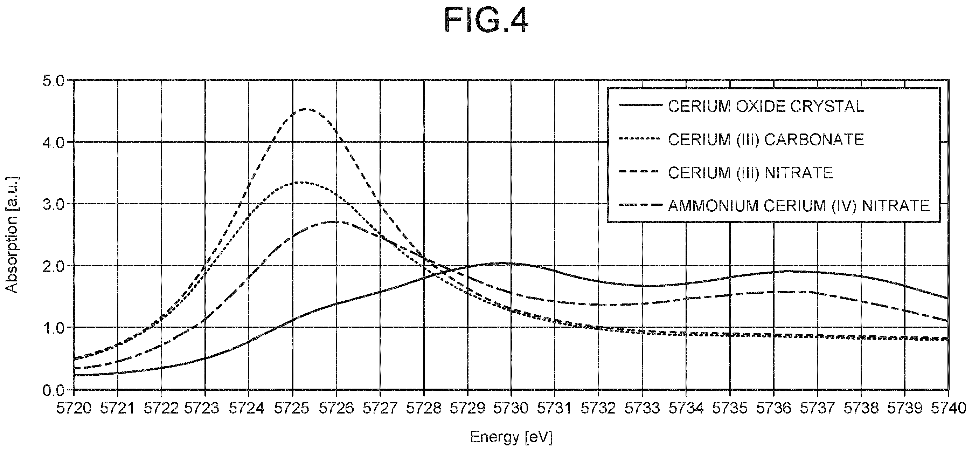

The XAFS observation was carried out with the same operations under the same conditions as those of the XAFS observations in Examples 1, 2, and 12, and Comparative Examples 2 and 5 as described above, except that the cerium oxide crystal, which is not the nanoparticle, cerium (III) carbonate, cerium (III) nitrate, or ammonium cerium (IV) nitrate, all three of which are cerium salts, were used. The Ce L3 edge XANES spectra of these are exhibited in FIG. 4. It was found that the cerium oxide crystal had the local maximum absorptions at 5729.810 eV and 5736.568 eV, cerium (III) carbonate had the local maximum absorption at 5725.161 eV, cerium (III) nitrate had the local maximum absorption at 5725.316 eV, and ammonium cerium (IV) nitrate had the local maximum absorptions at 5725.796 eV and 5736.105 eV; thus, it was found that none of the known cerium salts and the cerium compounds had the local maximum absorptions in the range of 5726 eV to 5729 eV and in the range of 5735 eV to 5739 eV.

### (Example 19) Dispersion Solution Containing Cerium Oxide Nanoparticles Using Boric Acid as Stabilizing Agent and Doped with 0.1 Mole of Cu (II) Compound

In Example 1, the reaction was carried out under the same conditions as in Example 1, except that after cerium nitrate was added, 23 µL of an aqueous 1 M copper (II) sulfate pentahydrate solution (0.1 mole relative to 1 mole of cerium nitrate hexahydrate) was added, to obtain a yellow-white colored dispersion solution containing the cerium oxide nanoparticles.

### (Example 20) Dispersion Solution Containing Cerium Oxide Nanoparticles Using Boric Acid as Stabilizing Agent and Doped with 0.05 Mole of Cu (II) Compound

In Example 19, the reaction was carried out under the same conditions as those of Example 19, except that the addition amount of the aqueous 1 M copper (II) sulfate pentahydrate solution was 11.5 µL (0.05 mole relative to 1 mole of cerium nitrate hexahydrate), to obtain a yellow-white colored dispersion solution containing the cerium oxide nanoparticles.

### (Example 21) Dispersion Solution Containing Cerium Oxide Nanoparticles Using Boric Acid as Stabilizing Agent and Doped with 0.01 Mole of Cu (II) Compound

In Example 19, the reaction was carried out under the same conditions as those of Example 19, except that the addition amount of the aqueous 1 M copper (II) sulfate pentahydrate solution was 2.3 µL (0.01 mole relative to 1 mole of cerium nitrate hexahydrate), to obtain an orange-colored dispersion solution containing the cerium oxide nanoparticles.

### (Example 22) Dispersion Solution Containing Cerium Oxide Nanoparticles Using Boric Acid as Stabilizing Agent and Doped with 0.1 Mole of Fe (II) Compound

In Example 1, the reaction was carried out under the same conditions as in Example 1, except that after cerium nitrate was added, 23 µL of an aqueous 1 M iron (II) sulfate heptahydrate solution (0.1 mole relative to 1 mole of cerium nitrate hexahydrate) was added, to obtain a yellow-white colored dispersion solution containing the cerium oxide nanoparticles.

### (Example 23) Dispersion Solution Containing Cerium Oxide Nanoparticles Using Boric Acid as Stabilizing Agent and Doped with 0.05 Mole of Fe (II) Compound

In Example 22, the reaction was carried out under the same conditions as those of Example 22, except that the addition amount of the aqueous 1 M iron (II) sulfate heptahydrate solution was 11.5 µL (0.05 mole relative to 1 mole of cerium nitrate hexahydrate), to obtain a yellow-white colored dispersion solution containing the cerium oxide nanoparticles.

### (Example 24) Dispersion Solution Containing Cerium Oxide Nanoparticles Using Boric Acid as Stabilizing Agent and Doped with 0.01 Mole of Fe (II) Compound

In Example 22, the reaction was carried out under the same conditions as those of Example 22, except that the addition amount of the aqueous 1 M iron (II) sulfate heptahydrate solution was 2.3 µL (0.01 mole relative to 1 mole of cerium nitrate hexahydrate), to obtain an orange-colored dispersion solution containing the cerium oxide nanoparticles.

### (Example 25) Dispersion Solution Containing Cerium Oxide Nanoparticles Using Boric Acid as Stabilizing Agent and Doped with 0.05 Mole of Fe (III) Compound

In Example 20, the reaction was carried out under the same conditions as those of Example 20, except that the aqueous 1 M copper (II) sulfate pentahydrate added was changed to the aqueous 1 M iron (III) chloride solution, to obtain an orange-colored dispersion solution containing the cerium oxide nanoparticles.

### (Example 26) Dispersion Solution Containing Cerium Oxide Nanoparticles Using Boric Acid as Stabilizing Agent and Doped with 0.05 Mole of Co Compound

In Example 20, the reaction was carried out under the same conditions as those of Example 20, except that the aqueous 1 M copper (II) sulfate pentahydrate added was changed to the aqueous 1 M cobalt (II) chloride solution, to obtain an orange-colored dispersion solution containing the cerium oxide nanoparticles.

### (Example 27) Dispersion Solution Containing Cerium Oxide Nanoparticles Using Boric Acid as Stabilizing Agent and Doped with 0.05 Mole of Zn Compound

In Example 20, the reaction was carried out under the same conditions as those of Example 20, except that the aqueous 1 M copper (II) sulfate pentahydrate added was changed to the aqueous 1 M zinc (II) nitrate solution, to obtain an orange-colored dispersion solution containing the cerium oxide nanoparticles.

### (Example 28) Measurement of Hydrodynamic Diameter of Cerium Oxide Nanoparticle Doped with Metal

The hydrodynamic diameter of the cerium oxide nanoparticle prepared in each of Examples 19 to 27 was measured by the dynamic light scattering (DLS) method. Water was used as the solvent for the measurement; the average particle diameter of the hydrodynamic diameter was obtained in terms of number conversion. The obtained values are listed in Table 6.

The average particle diameters were in the range of 3.1 to 45.2 nm, all of which were confirmed to be nanoparticles.

**Table 6**

| | Metal added | Metal content relative to 1 mole Ce [mol] | Quantification result by ICP analysis: Metal content relative to 1 mole Ce [mol] | Hydrodynamic diameter (nm) |
|---|---|---|---|---|
| Example 19 | Cu (II) | 0.1 | 0.0035 | 45.2 ± 9.3 |
| Example 20 | Cu (II) | 0.05 | 0.0017 | 35.7 ± 7.8 |
| Example 21 | Cu (II) | 0.01 | 0.00037 | 5.1 ± 1.1 |
| Example 22 | Fe (II) | 0.1 | 0.036 | 42.4 ± 8.5 |
| Example 23 | Fe (II) | 0.05 | 0.01 | 26.0 ± 5.4 |
| Example 24 | Fe (II) | 0.01 | 0.00045 | 3.7 ± 0.8 |
| Example 25 | Fe (III) | 0.05 | 0.00087 | 3.4 ± 0.8 |
| Example 26 | Co (II) | 0.05 | 0.00041 | 3.1 ± 0.7 |
| Example 27 | Zn (II) | 0.05 | 0.00027 | 3.4 ± 0.7 |

### (Example 29) Determination of Ce, Cu, Fe, Co, and Zn Using ICP Emission Spectrometry and ICP-MS

Each of the samples from Examples 19 to 27 were weighed into a Teflon (registered trademark) container, decomposed by heating with sulfuric acid, nitric acid, and hydrochloric acid, concentrated until generating a sulfuric acid white smoke, and then dissolved in a dilute aqua regia to obtain a solution with a certain volume. Ce in the resulting solution was determined by ICP emission spectrometry, and Cu, Fe, Co, and Zn were determined by ICP mass analysis method. The ICP emission spectrometer used was PS3520VDDII (manufactured by Hitachi High-Tech Science Corp.), and the ICP mass analysis instrument used was Agilent 8800 (manufactured by Agilent Technologies Inc.). The obtained values are listed in Table 6.

The actual addition amounts of the transition metals are in the range of 0.00027 to 0.036 per one mole of Ce; it was confirmed that all of them were nanoparticles.

### (Example 30) Antibacterial Test Against E. Coli

The pre-cultured E. coli in LB medium was suspended in a bacteria preparation solution (0.1% tryptone, 0.85% NaCl) to obtain a 10⁸ CFU/ml bacterial solution. This bacterial solution (0.1 ml) was mixed with 0.9 ml of each of the 1 mg/ml dispersion solutions of the cerium oxide nanoparticles prepared in Examples 1, 12, 20, 23, and 25 to 27; and then, the resulting mixture was allowed to leave at room temperature for 1 hour. Next, a dilution series was prepared from this mixture as a stock solution; then, this was inoculated on the LB agar medium to measure the number of colonies. The logarithmic reduction value of the number of colonies relative to the number of colonies before applying the cerium oxide nanoparticle is listed in Table 7.

From these results, it was found that the antibacterial activity values of the cerium oxide nanoparticles in Examples 1 and 12 were in the range of 2.2 to 2.3; thus, the antibacterial performance was confirmed in them. In addition, the antibacterial activity values of the cerium oxide nanoparticles in Examples 20, 23, and 25 to 27 are in the range of 3.0 to 5.6, indicating that doping of the metal species improved the antibacterial performance.

On the other hand, in the cerium oxide nanoparticle prepared in Comparative Example 1, the logarithmic reduction value was 0.64, indicating that this has a low antibacterial activity.

**Table 7**

| | Nanoparticle | Stabilizing agent | Metal added | Antibacterial activity value |
|---|---|---|---|---|
| Example 1 | Example 1 | Boric acid | None | 2.2 |
| Example 12 | Example 12 | Boric acid (Heating at 90°C) | None | 2.3 |
| Example 20 | Example 14 | Boric acid | Cu (II) | 4.8 |
| Example 23 | Example 17 | Boric acid | Fe (II) | 5.6 |
| Example 25 | Example 19 | Boric acid | Fe (III) | 3.2 |
| Example 26 | Example 19 | Boric acid | Co (II) | 3.0 |
| Example 27 | Example 20 | Boric acid | Zn (II) | 3.0 |
| Comparative Example 1 | Comparative Example 1 | Polyacrylic acid | None | 0.64 |

## Claims

1. A method for producing a cerium oxide nanoparticle, the method comprising
adding an oxidant to a solution comprising a boron compound represented by following general formula (I) and a cerium (III) ion:
BRₙ(OR')₃₋ₙ (I)
in formula (I), n represents an integer of 0 to 2, R represents any of an alkyl group having 1 to 4 carbon atoms, a phenyl group, and a tolyl group, and R' represents any of a hydrogen atom, an alkyl group having 1 to 4 carbon atoms, a phenyl group, and a tolyl group, and when a plurality of Rs or of R's are present, the plurality of Rs or of R's are optionally the same or different.

2. The method for producing a cerium oxide nanoparticle according to claim 1, wherein pH of the solution when the oxidant is added is 5 or more.

3. The method for producing a cerium oxide nanoparticle according to claim 1 or 2, wherein the boron compound represented by the general formula (I) is a boric acid, a boric acid ester, a boronic acid, a boronic acid ester, a borinic acid, a borinic acid ester, or a borate salt.

4. The method for producing a cerium oxide nanoparticle according to any one of claims 1 to 3, wherein 0.001 mole or more of boron relative to 1 mole of cerium element is included.

5. The method for producing a cerium oxide nanoparticle according to any one of claims 1 to 4, wherein a transition metal is included in the solution in an amount of 0.0001 to 0.3 mole, relative to one mole of the cerium (III) ion.

6. A cerium oxide nanoparticle, comprising a boron compound represented by following general formula (I), wherein
the cerium oxide nanoparticle has local maximum absorptions in 5726 eV to 5729 eV and in 5735 eV to 5739 eV in the XANES spectrum of the cerium oxide nanoparticle:
BRₙ(OR')₃₋ₙ (I)
in formula (I), n represents an integer of 0 to 2, R represents any of an alkyl group having 1 to 4 carbon atoms, a phenyl group, and a tolyl group, and R' represents any of a hydrogen atom, an alkyl group having 1 to 4 carbon atoms, a phenyl group, and a tolyl group, and when a plurality of Rs or of R's are present, the plurality of Rs or of R's are optionally same or different.

7. The cerium oxide nanoparticle according to claim 6, wherein the boron compound represented by the general formula (I) is a boric acid, a boric acid ester, a boronic acid, a boronic acid ester, a borinic acid, a borinic acid ester, or a borate salt.

8. The cerium oxide nanoparticle according to claim 6 or 7, wherein 0.001 mole or more of boron relative to 1 mole of cerium element is included.

9. The cerium oxide nanoparticle according to any one of claims 6 to 8, wherein 0.0001 mole or more of a transition metal relative to 1 mole of cerium element is included.

10. A dispersion solution comprising a cerium oxide nanoparticle obtained by the method according to any one of claims 1 to 5 or the cerium oxide nanoparticle according to any one of claims 6 to 9.

11. An oxidant comprising a cerium oxide nanoparticle obtained by the method according to any one of claims 1 to 5, the cerium oxide nanoparticle according to any one of claims 6 to 9, or the dispersion solution according to claim 10.

12. An antivirus agent comprising a cerium oxide nanoparticle obtained by the method according to any one of claims 1 to 5, the cerium oxide nanoparticle according to any one of claims 6 to 9, or the dispersion solution according to claim 10.

13. An antibacterial agent comprising a cerium oxide nanoparticle obtained by the method according to any one of claims 1 to 5, the cerium oxide nanoparticle according to any one of claims 6 to 9, or the dispersion solution according to claim 10.

## Patentansprüche

1. Verfahren zum Herstellen eines Ceroxid-Nanopartikels, wobei das Verfahren umfasst:
Hinzufügen eines Oxidationsmittels zu einer Lösung, die eine Bor-Verbindung, die durch die folgende allgemeine Formel (I) wiedergegeben wird, und ein Cer (III)-Ion umfasst,
BRₙ(OR')₃₋ₙ (I)
wobei in der Formel (I) n eine Ganzzahl von 0 bis 2 wiedergibt, R eine Alkylgruppe mit 1 bis 4 Kohlenstoffatomen, eine Phenylgruppe und eine Tolylgruppe wiedergibt und R' ein Wasserstoffatom, eine Alkylgruppe mit 1 bis 4 Kohlenstoffatomen, eine Phenylgruppe und eine Tolylgruppe wiedergibt, und wobei, wenn eine Vielzahl von Rs oder R's vorhanden sind, die Vielzahl von Rs oder R's optional gleich oder verschieden sind.

2. Verfahren zum Herstellen eines Ceroxid-Nanopartikels nach Anspruch 1, wobei der pH-Wert der Lösung, wenn das Oxidationsmittel hinzugefügt ist, 5 oder mehr beträgt.

3. Verfahren zum Herstellen eines Ceroxid-Nanopartikels nach Anspruch 1 oder 2, wobei die durch die allgemeine Formel (I) wiedergegebene Bor-Verbindung eine Borsäure, ein Borsäureester, eine Boronsäure, ein Boronsäurester, eine Borinsäure, ein Borinsäureester oder ein Boratsalz ist.

4. Verfahren zum Herstellen eines Ceroxid-Nanopartikels nach einem der Ansprüche 1 bis 3, wobei 0,001 mol oder mehr Boron relativ zu 1 mol des Cer-Elements enthalten sind.

5. Verfahren zum Herstellen eines Ceroxid-Nanopartikels nach einem der Ansprüche 1 bis 4, wobei ein Übergangsmetall in der Lösung mit einem Anteil von 0,0001 bis 0,3 mol relativ zu 1 mol des Cer (III)-Ions enthalten ist.

6. Ceroxid-Nanopartikel, das eine Bor-Verbindung, die durch die folgende allgemeine Formel (I) wiedergegeben wird, umfasst, wobei das Ceroxid-Nanopartikel lokale maximale Absorptionen in 5726 eV bis 5729 eV und in 5735 eV bis 5739 eV im XANES-Spektrum des Ceroxid-Nanopartikels aufweist:
BRₙ(OR')₃₋ₙ (I)
wobei in der Formel (I) n eine Ganzzahl von 0 bis 2 wiedergibt, R eine Alkylgruppe mit 1 bis 4 Kohlenstoffatomen, eine Phenylgruppe und eine Tolylgruppe wiedergibt und R' ein Wasserstoffatom, eine Alkylgruppe mit 1 bis 4 Kohlenstoffatomen, eine Phenylgruppe und eine Tolylgruppe wiedergibt, und wobei, wenn eine Vielzahl von Rs oder R's vorhanden sind, die Vielzahl von Rs oder R's optional gleich oder verschieden sind.

7. Ceroxid-Nanopartikel nach Anspruch 6, wobei die durch die allgemeine Formel (I) wiedergegebene Bor-Verbindung eine Borsäure, ein Borsäureester, eine Boronsäure, ein Boronsäurester, eine Borinsäure, ein Borinsäureester oder ein Boratsalz ist.

8. Ceroxid-Nanopartikel nach Anspruch 6 oder 7, wobei 0,001 mol oder mehr Boron relativ zu 1 mol des Cer-Elements enthalten sind.

9. Ceroxid-Nanopartikel nach einem der Ansprüche 6 bis 8, wobei 0,0001 mol oder mehr eines Übergangsmetalls relativ zu 1 mol des Cer-Elements enthalten sind.

10. Dispersionslösung, die ein Ceroxid-Nanopartikel, das durch das Verfahren gemäß einem der Ansprüche 1 bis 5 erhalten wird, oder das Ceroxid-Nanopartikel gemäß einem der Ansprüche 6 bis 9 umfasst.

11. Oxidationsmittel, das ein Ceroxid-Nanopartikel, das durch das Verfahren gemäß einem der Ansprüche 1 bis 5 erhalten wird, das Ceroxid-Nanopartikel gemäß einem der Ansprüche 6 bis 9 oder die Dispersionslösung gemäß Anspruch 10 umfasst.

12. Antivirales Mittel, das ein Ceroxid-Nanopartikel, das durch das Verfahren gemäß einem der Ansprüche 1 bis 5 erhalten wird, das Ceroxid-Nanopartikel gemäß einem der Ansprüche 6 bis 9 oder die Dispersionslösung gemäß Anspruch 10 umfasst.

13. Antibakterielles Mittel, das ein Ceroxid-Nanopartikel, das durch das Verfahren gemäß einem der Ansprüche 1 bis 5 erhalten wird, das Ceroxid-Nanopartikel gemäß einem der Ansprüche 6 bis 9 oder die Dispersionslösung gemäß Anspruch 10 umfasst.

## Revendications

1. Procédé de production d'une nanoparticule d'oxyde de cérium, le procédé comprenant
l'ajout d'un oxydant à une solution comprenant un composé de bore représenté par la formule générale suivante (I) et un ion cérium (III) :
BRₙ(OR')₃₋ₙ (I)
dans la formule (I), n représente un nombre entier de 0 à 2, R représente l'un quelconque parmi un groupe alkyle ayant de 1 à 4 atomes de carbone, un groupe phényle et un groupe tolyle, et R' représente l'un quelconque parmi un atome d'hydrogène, un groupe alkyle ayant de 1 à 4 atomes de carbone, un groupe phényle et un groupe tolyle, et lorsqu'une pluralité de R ou de R' sont présents, la pluralité de R ou de R' sont éventuellement identiques ou différents.

2. Procédé de production d'une nanoparticule d'oxyde de cérium selon la revendication 1, dans lequel le pH de la solution lorsque l'oxydant est ajouté est de 5 ou plus.

3. Procédé de production d'une nanoparticule d'oxyde de cérium selon la revendication 1 ou 2, dans lequel le composé de bore représenté par la formule générale (I) est un acide borique, un ester d'acide borique, un acide boronique, un ester d'acide boronique, un acide borinique, un ester d'acide borinique, ou un sel de borate.

4. Procédé de production d'une nanoparticule d'oxyde de cérium selon l'une quelconque des revendications 1 à 3, dans lequel 0,001 mole ou plus de bore par rapport à 1 mole d'élément cérium est incluse.

5. Procédé de production d'une nanoparticule d'oxyde de cérium selon l'une quelconque des revendications 1 à 4, dans lequel un métal de transition est inclus dans la solution en une quantité de 0,0001 à 0,3 mole, par rapport à une mole de l'ion cérium (III).

6. Nanoparticule d'oxyde de cérium, comprenant un composé de bore représenté par la formule générale (I) suivante,
dans laquelle
la nanoparticule d'oxyde de cérium présente des absorptions maximales locales entre 5 726 eV et 5 729 eV et entre 5 735 eV et 5 739 eV dans le spectre XANES de la nanoparticule d'oxyde de cérium :
BRₙ(OR')₃₋ₙ (I)
dans la formule (1), n représente un nombre entier de 0 à 2, R représente l'un quelconque parmi un groupe alkyle ayant de 1 à 4 atomes de carbone, un groupe phényle et un groupe tolyle, et R' représente l'un quelconque parmi un atome d'hydrogène, un groupe alkyle ayant de 1 à 4 atomes de carbone, un groupe phényle et un groupe tolyle, et lorsqu'une pluralité de R ou de R' sont présents, la pluralité de R ou de R' sont éventuellement identiques ou différents.

7. Nanoparticule d'oxyde de cérium selon la revendication 6, dans laquelle le composé de bore représenté par la formule générale (I) est un acide borique, un ester d'acide borique, un acide boronique, un ester d'acide boronique, un acide borinique, un ester d'acide borinique, ou un sel de borate.

8. Nanoparticule d'oxyde de cérium selon la revendication 6 ou 7, dans laquelle 0,001 mole ou plus de bore par rapport à 1 mole d'élément cérium est incluse.

9. Nanoparticule d'oxyde de cérium selon l'une quelconque des revendications 6 à 8, dans laquelle 0,0001 mole ou plus d'un métal de transition par rapport à 1 mole d'élément cérium est incluse.

10. Solution de dispersion comprenant une nanoparticule d'oxyde de cérium obtenue par le procédé selon l'une quelconque des revendications 1 à 5 ou la nanoparticule d'oxyde de cérium selon l'une quelconque des revendications 6 à 9.

11. Oxydant comprenant une nanoparticule d'oxyde de cérium obtenue par le procédé selon l'une quelconque des revendications 1 à 5, la nanoparticule d'oxyde de cérium selon l'une quelconque des revendications 6 à 9, ou la solution de dispersion selon la revendication 10.

12. Agent antiviral comprenant une nanoparticule d'oxyde de cérium obtenue par le procédé selon l'une quelconque des revendications 1 à 5, la nanoparticule d'oxyde de cérium selon l'une quelconque des revendications 6 à 9, ou la solution de dispersion selon la revendication 10.

13. Agent antibactérien comprenant une nanoparticule d'oxyde de cérium obtenue par le procédé selon l'une quelconque des revendications 1 à 5, la nanoparticule d'oxyde de cérium selon l'une quelconque des revendications 6 à 9, ou la solution de dispersion selon la revendication 10.
